(19) Europäisches Patentamt
European Patent Office
Office européen des brevets

(11) EP 3 135 766 B1

(12) # EUROPEAN PATENT SPECIFICATION

(45) Date of publication and mention of the grant of the patent:
**02.12.2020 Bulletin 2020/49**

(21) Application number: **15783801.2**

(22) Date of filing: **24.04.2015**

(51) Int Cl.:
***C12N 15/115*** *(2010.01)* ***A61K 31/7105*** *(2006.01)*
***A61K 45/00*** *(2006.01)* ***A61K 48/00*** *(2006.01)*
***A61P 11/00*** *(2006.01)* ***A61P 43/00*** *(2006.01)*

(86) International application number:
**PCT/JP2015/062561**

(87) International publication number:
**WO 2015/163458 (29.10.2015 Gazette 2015/43)**

(54) **APTAMER FOR BONDING TO AUTOTAXIN AND INHIBITING BIOLOGICAL ACTIVITY OF AUTOTAXIN, AND USE FOR SAME**

APTAMER ZUR BINDUNG AN AUTOTAXIN UND HEMMUNG DER BIOLOGISCHEN AKTIVITÄT VON AUTOTAXIN SOWIE VERWENDUNG DAFÜR

APTAMÈRE UTILISÉ POUR SE LIER À L'AUTOTAXINE ET POUR INHIBER L'ACTIVITÉ BIOLOGIQUE DE L'AUTOTAXINE, ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.04.2014 JP 2014090755**

(43) Date of publication of application:
**01.03.2017 Bulletin 2017/09**

(73) Proprietor: **RIBOMIC INC.**
**Minato-ku**
**Tokyo 108-0071 (JP)**

(72) Inventor: **IKEDA, Hisako**
**Tokyo 108-0071 (JP)**

(74) Representative: **J A Kemp LLP**
**14 South Square**
**Gray's Inn**
**London WC1R 5JJ (GB)**

(56) References cited:
**WO-A1-2013/047844** **WO-A1-2013/186857**
**JP-A- 2005 527 614** **JP-A- 2013 524 251**

- **SHIN'ICHI OKUDAIRA ET AL.: 'Hai Sen'isho ni Okeru Lysophosphatidic Acid Sansei Koso Autotaxin no Kino Kaiseki' PROCEEDINGS OF JAPANESE CONFERENCE ON THE BIOCHEMISTRY OF LIPIDS vol. 51, 10 July 2009, pages 254 - 257, XP008185168**
- **TAGER , A.M.: 'Autotaxin emerges as a therapeutic target for idiopathic pulmonary fibrosis: limiting fibrosis by limiting lysophosphatidic acid synthesis.' AM. J. RESPIR. CELL MOL. BIOL. vol. 47, no. 5, 2012, pages 563 - 565, XP055232663**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

[Technical Field]

The present invention relates to an aptamer having a binding activity to and an inhibitory activity against autotaxin and a utilization method thereof and the like.

[Background Art]

Autotaxin is a secretory protein identified as a molecule that promotes motility of melanoma cells. It belongs to the Enpp (ectonucleotide pyrophosphatase/phosphodiesterase) family proteins and also known as Enpp2. It has a phosphodiesterase activity and is involved in extracellular nucleotide metabolism. It also has a Lysophospholipase D activity (LysoPLD activity), and is also an enzyme that degrades lysophosphatidylcholine (LPC) into lysophosphatidic acid (LPA) and choline. Produced LPA shows various physiological activities of a lipid mediator, such as cellular motility activation, cell proliferation, angiogenesis and the like. LPA is said to be involved in the growth, metastasis and the like of cancer cells, and many studies of LPA have been made. Also, there are many reports on increased expression and activity of autotaxin, which is an LPA producing enzyme, in the blood and ascites of cancer patients.

Recently, a fibrosis suppressive effect by LPA receptor LPA1 knocked-out mouse and LPA1 inhibitors in a pulmonary fibrosis model by bleomycin induction has been reported, thus suggesting relation between LPA and pulmonary fibrosis, and autotaxin as an LPA producing enzyme is drawing attention as to the relation with pulmonary fibrosis.

Among them is a report that an anti-autotaxin monoclonal antibody has a prophylactic and/or treatment effect on interstitial pneumonia and/or pulmonary fibrosis. In addition, a fibrosis suppressive effect of a small-molecule inhibitor of autotaxin in a pulmonary fibrosis model by bleomycin induction has been reported. All these reports show that autotaxin is present in the alveolar lavage fluid of idiopathic pulmonary fibrosis patients, and the concentration and activity thereof are high as compared to healthy individuals.

Idiopathic pulmonary fibrosis is a disease showing extremely poor prognosis as evidenced by a five-year survival rate of 30%. While the mechanism thereof contains many unclear aspects, it is generally understood that damage on alveoli and the like causes excessive action of the tissue repair mechanism, and abnormal growth of fibroblasts and excessive production of connective tissue protein occur in pulmonary interstitium. At present, steroids, immunosuppressants and the like are used for a global standard treatment. In 2008, for the first time in the world, Pirespa (general name: pirfenidone) was approved in Japan as a therapeutic drug for idiopathic pulmonary fibrosis, and the effectiveness thereof and the like are being studied in clinical situations. However, the action mechanism thereof contains many unclear aspects such as what is the target of Pirespa and the like.

Aptamer means a nucleic acid that specifically binds to a target molecule (protein, sugar chain, hormone etc.). It binds to a target molecule due to a three-dimensional structure of a single strand RNA (or DNA). To obtain same, a screening method called a SELEX method (Systematic Evolution of Ligands by Exponential Enrichment) is used (patent documents 1-3). An aptamer obtained by the SELEX method has a chain length of about 80 nucleotides, which is thereafter shortened with a physiological inhibitory activity of the target molecule as an index. It is further modified chemically to improve in vivo stability, thus optimizing same as a pharmaceutical product.

Aptamers show high binding property to the target molecule, and the affinity thereof is often high compared to antibodies having a similar function. Aptamers are unlikely to undergo immune elimination, and adverse reactions characteristic of antibodies, such as antibody-dependent cell-mediated cytotoxicity (ADCC) and complement-dependent cytotoxicity (CDC), do not occur easily with the use of aptamers. From the aspect of delivery, since aptamers are about 1/10 of antibody in molecular size, tissue transfer occurs easily and the delivery of a drug to the object site is easier. Some molecular targeting drugs having a low molecular weight are poorly soluble and require optimization for formulation thereof. Since aptamers have high water-solubility, they are advantageous in such aspect.

Furthermore, since aptamers are produced by chemical synthesis, cost-cutting is possible by large-scale production. Besides these, long-term preservation stability and thermal· solvent tolerance are also superior characteristics of the aptamers. On the other hand, the blood half-lives of aptamers are generally shorter than those of antibodies; however, this property is sometimes advantageous in view of toxicity.

In December 2004, the world's first RNA aptamer drug, Macugen, was approved in USA as a therapeutic drug for age-related macular degeneration, and the application of RNA aptamer to a therapeutic drug, a diagnostic agent or a reagent is attracting attention, and the drug is expected to be a next-generation pharmaceutical product.

[Document List]

[patent documents]

[patent document 1] WO 91/19813
[patent document 2] WO 94/08050
[patent document 3] WO 95/07364

[SUMMARY OF THE INVENTION]

[Problems to be Solved by the Invention]

The present invention is directed to providing an aptamer that binds to an autotaxin and the use thereof in the treatment or prophylaxis of diseases involving organ or tissue fibrosis.

[Means of Solving the Problems]

The present inventors investigated diligently to solve the problem described above and succeeded in preparing an aptamer of good quality for autotaxin, which resulted in the completion of the present invention.
Accordingly, the present invention provides the following invention.

[1] An aptamer that binds to an autotaxin, which comprises a nucleotide sequence represented by the following formula GWAACAGGUUUUGCU (SEQ ID NO: 42)
wherein W is A or U (provided that uracil is optionally thymine), and which is the following (a) or (b):

(a) an aptamer wherein, in the nucleotides contained in the aptamer,

(i) the 2'-position of the ribose of each pyrimidine nucleotide is a fluorine atom,
(ii) the 2'-position of the ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

[2] The aptamer of [1], wherein W is A.
[3] The aptamer of [1] or [2], which comprises the nucleotide sequence shown in SEQ ID NO: 16.
[4] The aptamer of [1], which comprises the nucleotide sequence shown in SEQ ID NO: 10 wherein, in SEQ ID NO: 10, 1 - several nucleotides are further substituted, inserted or added, and which is the following (a) or (b):

(a) an aptamer wherein, in the substituted, inserted or added nucleotides,

(i) the 2'-position of the ribose of each pyrimidine nucleotide is a fluorine atom,
(ii) the 2'-position of the ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group

and a fluorine atom.

[5] The aptamer of [1], which comprises a nucleotide sequence of any of the following (A), (B) and (C):

(A) a nucleotide sequence selected from SEQ ID NOs: 4, 8, 14 - 20, 24, 29, 30, 32, 40, 41, 44, 45, 47, 48 and 50 - 53 (provided that uracil is optionally thymine);
(B) a nucleotide sequence selected from SEQ ID NOs: 4, 8, 14 - 20, 24, 29, 30, 40, 44, 45, 47, 48 and 50 - 53 (provided that uracil is optionally thymine), wherein 1 - several nucleotides are substituted, deleted, inserted or added nucleotide sequence;
(C) a nucleotide sequence having identity of not less than 60% with a nucleotide sequence selected from SEQ ID NOs: 4, 8, 14 - 20, 24, 29, 30, 40, 44, 45, 47, 48 and 50 - 53 (provided that uracil is optionally thymine); which is the following (a) or (b):

(a) an aptamer wherein, in the nucleotides contained in the aptamer,

(i) the 2'-position of the ribose of each pyrimidine nucleotide is a fluorine atom,
(ii) the 2'-position of the ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

[6] The aptamer of [1], which comprises a nucleotide sequence of any of the following (A'), (B') and (C'):

(A') a nucleotide sequence shown in SEQ ID NO: 16 (provided that uracil is optionally thymine);
(B') a nucleotide sequence shown in SEQ ID NO: 16 (provided that uracil is optionally thymine) (excluding a sequence shown by GAAACAGGUUUUGCU (SEQ ID NO: 10)), wherein 1 - 7 nucleotides are substituted, deleted, inserted or added;
(C') a nucleotide sequence having identity of not less than 70% with a nucleotide sequence shown in SEQ ID NO: 16 (provided that uracil is optionally thymine) (excluding a sequence shown by GAAACAGGUUUUGCU (SEQ ID NO: 10); which is the following (a) or (b):

(a) an aptamer wherein, in the nucleotides contained in the aptamer,

(i) the 2'-position of the ribose of each pyrimidine nucleotide is a fluorine atom,
(ii) the 2'-position of the ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

[7] The aptamer of any one of [1] - [6], which has a base length of not less than 23.
[8] The aptamer of any one of [1] - [7], wherein at least one nucleotide is modified or alteration.
[9] The aptamer of [8], which is modified by inverted dT or polyethylene glycol.
[10] The aptamer of [9], wherein the inverted dT or polyethylene glycol binds to 5'-terminus or 3'-terminus of the aptamer.
[11] The aptamer of any of [1] - [10], wherein at least one phosphate group contained in the aptamer is phosphorothioated or phosphorodithioated.

[12] The aptamer of [1], which comprises a nucleotide sequence shown in SEQ ID NO: 16, wherein, in the nucleotide sequence, at least one phosphoric acid group in a sequence shown by GAAACAGGUUUUGCU (SEQ ID NO: 10) is phosphorothioated or phosphorodithioated, and the 5'-terminus or 3'-terminus of the aptamer is modified by inverted dT or polyethylene glycol, respectively, and which is the following (a) or (b):

(a) an aptamer wherein, in the nucleotides contained in the aptamer,

(i) the 2'-position of the ribose of each pyrimidine nucleotide is a fluorine atom,
(ii) the 2'-position of the ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

[13] A complex comprising the aptamer of any of [1] - [12] and a functional substance.
[14] A medicament comprising the aptamer of any of [1] - [12], or the complex of [13].
[15] The aptamer according to any one of [1]-[12], or the complex of [13], for use in the treatment or prophylaxis of diseases involving organ or tissue fibrosis.

[Effect of the Invention]

The aptamer and complex of the present invention can be useful as, for example, a medicament or a diagnostic agent or a reagent for various diseases caused by autotaxin such as fibrosis, cancer and the like. The aptamer and complex of the present invention can also be useful for purification and concentration of autotaxin, as well as detection and quantification of autotaxin.

[Brief Description of the Drawings]

Fig. 1 shows secondary structures of aptamers shown in SEQ ID NOs: 4 and 16, which are predicted by the MFOLD program. The nucleotides of the sequence shown in SEQ ID NO: 10 are shown with circled characters.
Fig. 2 shows binding of the aptamer shown in SEQ ID NO: 4 to an autotaxin. 30N is an RNA pool containing random sequences of 30 nucleotides. As a capture molecule, an aptamer or 30N as a negative control was immobilized, and human autotaxin was flown as an analyte. The measurement was performed using Biacore T100 manufactured by GE Healthcare.
Fig. 3 shows that an aptamer shown in SEQ ID NO: 16(23) does not have a binding activity to FGF2.
Fig. 4 shows the effect of an autotaxin aptamer administration on the hydroxyproline level of the lung in bleomycin-induced pulmonary fibrosis model mouse. The left lung was isolated from a group administered with, from the next day of bleomycin administration, two doses of autotaxin aptamer (SEQ ID NO: 16(49)) every day, and the vehicle administration group, after completion of the administration, hydroxyproline amount per lung weight was measured and compared. The control group is a non-treated (bleomycin non-administrated) mouse.
Fig. 5 shows the effect of an autotaxin aptamer administration on clinical finding of fibrosis in bleomycin-induced pulmonary fibrosis model mouse. The lung was isolated from a group administered with, from the next day of bleomycin administration, two doses of autotaxin aptamer (SEQ ID NO: 16(49)) every day, and the vehicle administration group, after completion of the administration, and tissue sections were prepared and fibrosis was evaluated by the clinical scores. The control group is a non-treated (bleomycin non-administrated) mouse.

[Description of Embodiments]

The present invention provides an aptamer having a binding activity to an autotaxin (hereinafter to be also referred to as the aptamer of the present invention). The aptamer of the present invention can inhibit the activities of autotaxin.

An aptamer refers to a nucleic acid molecule having a binding activity to a particular target molecule. The aptamer can inhibit the activity of a particular target molecule by binding to the particular target molecule. The aptamer of the present invention has a binding activity to an autotaxin, and can inhibit the activity of autotaxin. The aptamer of the present invention may be an RNA, a DNA, a modified nucleic acid or a mixture thereof. The aptamer of the present invention can also be in a linear or circular form.

Autotaxin (EC.3.1.4.39) is a glycoprotein present in the blood, and is an enzyme that degrades lysophosphatidylcholine (LPC) into lysophosphatidic acid (LPA) and choline. The aptamer of the present invention can exhibit an inhibitory activity against autotaxin derived from any mammals. Such mammals include primates (e.g., human, monkey), rodents (e.g., mouse, rat, guinea pig, hamster), and companion animals, domestic animals and working animals (e.g., dog, cat, horse, bovine, goat, sheep, swine), preferably human.

As for human autotaxin, 4 isotypes of α, β, γ, δ have been reported. In the present invention, human autotaxin particularly means β type. The amino acid sequence of human β-autotaxin is identified by NCBI accession number NP_001035181, and the human autotaxin also includes a partial protein having a substantially equivalent LPA synthesis activity and a mutated protein wherein a part of the amino acid is substituted, deleted, added or inserted.

The aptamer of the present invention binds to an autotaxin in a physiological buffer. While the buffer is not particularly limited, one having pH about 5.0 - 10.0 is preferably used. Examples of such buffer include below-mentioned solution A (see Example 1). The aptamer of the present invention binds to an autotaxin with the strength of a level detectable by any test shown below.

Biacore T100 manufactured by GE Healthcare is used for the measurement of binding strength. In one measurement method, an aptamer is first immobilized on a sensorchip. The immobilization amount is set to about 1500RU. An autotaxin solution as an analyte prepared to 0.020 μM is injected by 20 μL, and binding of the autotaxin to the aptamer is detected. Using RNA containing a random nucleotide sequence consisting of 30 nucleotides as a negative control, when the autotaxin significantly strongly bound to the aptamer as compared to the control RNA, the aptamer can be judged to have a binding ability to autotaxin.

In another measurement method, an autotaxin is first immobilized on a sensorchip. The immobilization amount is set to about 2700RU. An aptamer solution as an analyte prepared to 0.30 μM is injected by 20 μL, and binding of the aptamer to the autotaxin is detected. Using RNA containing a random nucleotide sequence consisting of 30 nucleotides as a negative control, when the aptamer significantly strongly bonded to the autotaxin as compared to the control RNA, the aptamer can be judged to have a binding ability to autotaxin.

The inhibitory activity against an autotaxin means an inhibitory ability against any activity that the autotaxin has. While autotaxin has a phosphodiesterase activity to cleave phosphodiester bond by hydrolysis, such activity is inhibited. An acceptable substrate for enzyme activity is not limited to a phosphodiester bond-containing substance (e.g., ATP and the like) present in vivo, and includes a substrate wherein a compound containing same is added with a chromogenic substance or a fluorescent substance. The chromogenic substance and fluorescent substance are known to those of ordinary skill in the art. Also, autotaxin has a lysophospholipase D activity. By this activity, lysophosphatidic acid (LPA) is mainly produced by cleaving the bond on the side opposite from the glycerol backbone of phosphodiester of lysophospholipid. Inhibition of autotaxin activity also includes suppression of the production.

A substrate of autotaxin refers to a substance having a phosphodiester bond to be cleaved by hydrolysis by autotaxin. As a substrate of autotaxin present in vivo, lysophosphatidylcholine (LPC) and sphingosylphosphorylcholine (SPC) are known. The substrate of autotaxin in the present specification also includes LPC and SPC having various carbon chain lengths and degrees of unsaturation, and those added with a chromogenic substance or a fluorescent substance.

Whether an aptamer inhibits the enzyme activity of autotaxin can be evaluated, for example, by the following test. As a substrate of autotaxin, phosphodiester bond-containing synthetic substrate p-nitrophenyl thymidine 5'-monophosphate (pNP-TMP) (SIGMA) is used. A phosphodiester bond is cleaved by hydrolysis, and p-nitrophenol is liberated. The p-nitrophenol develops a yellow color, and the color is detected. For the assay, a 96-well plate (96-Well EIA/RIA Polystyrene Plates, Costar) is used, and the amount of the reaction mixture is 200 μL. Nucleic acid is prepared in solution A (see below-mentioned Example 1) (100 μL), 10 mM pNP-TMP (20 μL) adjusted in the reaction mixture A is added, and the mixture is stirred well and heated at 37°C for 5 min. On the other hand, 6 ng of autotaxin (Recombinant Human, manufactured by R&D) diluted with solution A is prepared (80 μL), and heated at 37°C for 5 min. After heating, they are mixed to start an enzyme reaction. The final autotaxin concentration in the reaction solution is 0.3 nM, and the final substrate concentration is 1 mM. A plate containing the reaction mixture is heated at 37°C for 24 hr, placed in a microplate reader SpectraMax190 (manufactured by Molecular Devices) and the absorbance is determined at wavelength 405 nm. The absorbance when nucleic acid is not added as 100% (A0), an enzyme activity rate is determined from the absorbance (A) of each test substance and according to the following formula.

$$\text{Enzyme activity rate} = (A/A0) \times 100$$

The concentration ($IC_{50}$) of an inhibitor necessary for inhibiting the enzyme activity by 50% is determined. An aptamer having an $IC_{50}$ value of not more than 0.10 $\mu$M is judged to be an aptamer having a superior inhibitory activity.

The aptamer of the present invention is not particularly limited as long as it binds to any part of an autotaxin. The aptamer of the present invention is not particularly limited as long as it binds to any part of autotaxin and can inhibit the activity thereof.

The length of the aptamer of the present invention is not particularly limited, and can usually be not more than about 200 nucleotides. For example, it may be not more than about 100 nucleotides, preferably not more than about 50 nucleotides, more preferably not more than about 40 nucleotides, most preferably not more than about 30 nucleotides. When the total number of nucleotides is smaller, chemical synthesis and mass-production will be easier, and there is a major advantage in terms of cost. It is also thought that chemical modification is easy, stability in the body is high, and toxicity is low. The length of the lower limit of the aptamer of the present invention is not particularly limited as long as it includes the common sequence shown in SEQ ID NO: 53 (GWAACAGGUYYYGCU; W is A or U, and Y is U or C, provided that uracil is optionally thymine), and the common sequence can have a desired secondary structure (secondary structure of the below-mentioned formula (I') or (II)), the aptamer length may be, for example, not less than 15 nucleotides, preferably not less than 20 nucleotides, more preferably not less than 23 nucleotides. In a particularly preferable embodiment, the length of the aptamer of the present invention is 23-50 nucleotides, or 23-40 nucleotides.

In a preferable one embodiment, the aptamer of the present invention comprises a nucleotide sequence represented by the following formula
GWAACAGGUUUUGCU (SEQ ID NO: 42)
wherein W is A or U (provided that uracil is optionally thymine) (hereinafter to be also referred to as aptamer (I) of the present invention).

Each nucleotide contained in aptamer (I) of the present invention is the following (a) or (b):

(a)

(i) the 2'-position of the ribose of each pyrimidine nucleotide is a fluorine atom,
(ii) the 2'-position of the ribose of each purine nucleotide is a hydroxy group;

(b)

(i) the fluorine atom at the 2'-position of the ribose of each pyrimidine nucleotide is independently unsubstituted, or substituted by an atom or group selected from the group consisting of a hydrogen atom, a hydroxy group, an alkoxy group (e.g., methoxy group), an acyloxy group (e.g., acetyloxy group) and an amino group (e.g., $-NH_2$ group), preferably a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a fluorine atom or an alkoxy group (e.g., methoxy group), an acyloxy group (e.g., acetyloxy group) and an amino group (e.g., $-NH_2$ group), preferably a hydrogen atom, a methoxy group and a fluorine atom.

In the formula: GWAACAGGUUUUGCU (SEQ ID NO: 42), W is preferably A.

A sequence shown by the formula: GWAACAGGUUUUGCU (SEQ ID NO: 42) can form a potential secondary structure represented by the formula (I):

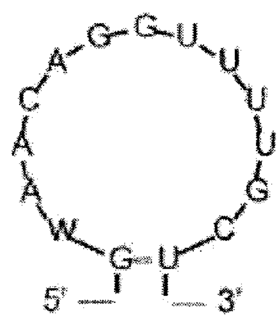


(I)

or the formula (II):

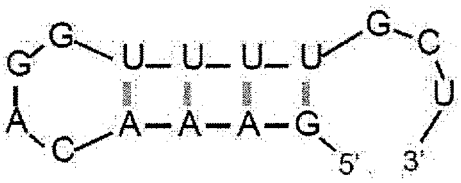

(II) .

In the aptamer of the present invention, a sequence part of GWAACAGGUUUUGCU (SEQ ID NO: 42) wherein W is A or U) can have the above-mentioned structure. Due to this structure, various activities to autotaxin (binding activity to autotaxin, autotaxin inhibitory activity etc.) are considered to be afforded.

In addition, a stem structure can be preferably formed by an interaction between the sequences following the 5'-terminus and 3'-terminus in the above-mentioned structure. For the aptamer of the present invention to show various activities (binding activity to autotaxin, autotaxin inhibitory activity etc.), it is desirable to have the above-mentioned potential secondary structure followed by a subsequent stem structure. Having the stem structure, the aptamer is stabilized to have a strong activity. While a stem structure can be formed by complementary base pairs (also including G=U base pairs in addition to Watson-Crick base pairs), the number of base pairs (stem length) is not particularly limited. Preferably, the stem length is not less than 4 base pairs. While the upper limit is not particularly limited, for example, it is not more than 18 base pairs, preferably not more than 11 base pairs. In the stem structure, even when base pairs are not formed in a part thereof, the aptamer activity is maintained as long as a stem structure is constituted as a whole. Therefore, the stem structure part can contain substitution, deletion, insertion, addition and the like of nucleotides as long as the structure thereof is maintained. A specific example of the stem structure is shown in, for example, Fig. 1.

As a preferable example of the nucleotide sequence wherein a stem structure can be formed by an interaction between the sequences following the 5'-terminus and 3'-terminus of the sequence shown by the formula: GWAACAGGUUUUGCU (SEQ ID NO: 42) wherein W is A or U, a nucleotide sequence shown in SEQ ID NO: 16 can be mentioned. A potential secondary structure that the nucleotide sequence shown in SEQ ID NO: 16 can take is shown in Fig. 1.

In the aptamer (I) of the present invention, 1 - several (e.g., 1 - 4, preferably 1 - 3, more preferably 1 - 2, particularly preferably 1) nucleotide may be substituted, inserted or added in the nucleotide sequence shown in SEQ ID NO: 42 as long as the binding activity to autotaxin and inhibitory activity against autotaxin activity (enzyme activity of autotaxin etc.) are retained. While the position of substitution or insertion of nucleotide is not particularly limited, for example, when a nucleotide is substituted, at least one U of the 10th - 12th UUU from the 5'-terminus of a nucleotide sequence shown in SEQ ID NO: 10 is preferably substituted by other nucleotide, preferably a nucleotide other than A, more preferably C.

Therefore, preferably, the aptamer (I) of the present invention can contain a nucleotide sequence shown by the formula: GWAACAGGUYYYGCU wherein W is A or U, and Y is U or C) (SEQ ID NO: 49). In the formula, W is preferably A, and YYY is preferably UUU, UUC, UCU or CUU. In the nucleotide sequence shown in SEQ ID NO: 42, when the 6th A from the 5'-terminus is substituted, it is preferably substituted by a nucleotide other than U, more preferably the 6th A from the 5'-terminus is not substituted.

In the above, the nucleotide to be substituted, inserted or added is the following (a) or (b):

(a)

(i) the 2'-position of the ribose of each pyrimidine nucleotide is a fluorine atom,
(ii) the 2'-position of the ribose of each purine nucleotide is a hydroxy group;

(b)

(i) the fluorine atom at the 2'-position of the ribose of each pyrimidine nucleotide is independently unsubstituted, or substituted by an atom or group selected from the group consisting of a hydrogen atom, a hydroxy group, an alkoxy group (e.g., methoxy group), an acyloxy group (e.g., acetyloxy group) and an amino group (e.g., $-NH_2$ group), preferably a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a fluorine atom or an alkoxy group (e.g., methoxy group), an acyloxy group (e.g., acetyloxy group) and an amino group (e.g., $-NH_2$ group), preferably a hydrogen atom, a methoxy group and a fluorine atom.

Alternatively, in another preferable embodiment, the aptamer of the present invention is an aptamer of any of the following (A) - (C):

(A) an aptamer containing a nucleotide sequence selected from SEQ ID NOs: 4, 8, 14 - 20, 24, 29, 30, 32, 40, 41, 44 - 48 and 50 - 53 (provided that uracil is optionally thymine);
(B) an aptamer containing a nucleotide sequence selected from SEQ ID NOs: 4, 8, 14 - 20, 24, 29, 30, 40, 44, 45, 47, 48 and 50 - 53 (provided that uracil is optionally thymine), wherein 1 - several nucleotides are substituted, deleted, inserted or added nucleotide sequence;
(C) an aptamer containing a nucleotide sequence having identity of not less than 60% (preferably not less than 70%, more preferably not less than 75%, further preferably not less than 80%, further more preferably not less than 85%, particularly preferably not less than 90%, most preferably not less than 95%) with a nucleotide sequence selected from SEQ ID NOs: 4, 8, 14 - 20, 24, 29, 30, 40, 44, 45, 47, 48 and 50 - 53 (provided that uracil is optionally thymine);

(provided that the aptamers of the above-mentioned (B) and (C) can bind to autotaxin to inhibit the activities of autotaxin (enzyme activity etc. of autotaxin)) (hereinafter to be also referred to as the aptamer (II) of the present invention).

Each nucleotide sequence shown in SEQ ID NO: 4, 8, 14 - 20, 24, 29, 30, 40, 44, 45, 47, 48 or 50 - 53 of the above-mentioned (B) and (C) contains a sequence shown by the formula: GWAACAGGUUUUGCU (SEQ ID NO: 42) wherein W is A or U) as a common sequence.

In the above-mentioned (b), the number of nucleotides to be substituted, deleted, inserted or added is not particularly limited as long as the aptamer can bind to an autotaxin and inhibit activity of autotaxin (enzyme activity etc. of autotaxin). For example, it may be not more than about 30, preferably not more than about 20, more preferably not more than about 10, further preferably not more than 5, most preferably 4, 3, 2 or 1. When the common sequence shown in SEQ ID NO: 10 contains substitution, insertion or addition of nucleotides, it is preferable to contain mutations similar to those in the above-mentioned aptamer (I) of the present invention. More preferably, when the common sequence contains substitution of nucleotide, the aptamer of the above-mentioned (B) contains a nucleotide sequence shown by the formula: GWAACAGGUYYYGCU wherein W is A or U, and Y is U or C) (SEQ ID NO: 49). In the formula, W is preferably A, and YYY is preferably UUC, UCU or CUU. In the nucleotide sequence shown in SEQ ID NO: 10, when the 6th A from the 5'-terminus is substituted, it is preferably substituted by a nucleotide other than U, more preferably the 6th A from the 5'-terminus is not substituted.

In the above-mentioned (c), the "identity" means a ratio (%) of the same nucleotide residues to all overlapping nucleotide residues in an optimal alignment (preferably, the algorithm can take into consideration an introduction of a gap into one or both of the sequences for optimal alignment) when two nucleotide sequences are aligned using a mathematical algorithm known in the art.

In the present specification, the identity of nucleotide sequence can be calculated by, for example, aligning two nucleotide sequences under the following conditions (gap opening =5 penalties; gap extension =2 penalties; x_drop off =50; expectancy =10; filtering=ON) by using homology calculation algorithm NCBI BLAST-2 (National Center for Biotechnology Information Basic Local Alignment Search Tool).

In the above-mentioned aptamer (C), the identity with the common sequence shown in SEQ ID NO: 42 is not less than 80%, preferably not less than 85%, more preferably not less than 90%, and particularly preferably, the aptamer contains a nucleotide sequence shown by the formula: GWAACAGGUYYYGCU wherein W is A or U, and Y is U or C) (SEQ ID NO: 49). In the formula, W is preferably A, and YYY is preferably UUC, UCU or CUU. In the nucleotide sequence shown in SEQ ID NO: 10, when the 6th A from the 5'-terminus is substituted, it is preferably substituted by a nucleotide other than U, more preferably the 6th A from the 5'-terminus is not substituted.

In a particularly preferable embodiment, the aptamer (II) of the present invention is an aptamer containing a nucleotide sequence of the following (A'), (B') or (C'):

(A') an aptamer containing a nucleotide sequence shown in SEQ ID NO: 16 (provided that uracil is optionally thymine);
(B') an aptamer containing a nucleotide sequence shown in SEQ ID NO: 16 (provided that uracil is optionally thymine) (excluding a sequence shown by GAAACAGGUUUUGCU (SEQ ID NO: 10)), wherein 1 - 7 nucleotides are substituted, deleted, inserted or added;
(C') an aptamer containing a nucleotide sequence having identity of not less than 70% (preferably not less than 75%, more preferably not less than 80%, further preferably not less than 85%, particularly preferably not less than 90%, most preferably not less than 95%) with a nucleotide sequence shown in SEQ ID NO: 16 (provided that uracil is optionally thymine) (excluding a sequence shown by GAAACAGGUUUUGCU (SEQ ID NO: 10));

(provided that the aptamers of the above-mentioned (B') and (C') can bind to autotaxin to inhibit the activities of autotaxin (enzyme activity etc. of autotaxin)).

In the above-mentioned (B'), the number of nucleotides to be substituted, deleted, inserted or added may be preferably not more than 5, more preferably 4, further preferably 3, particularly preferably 2 or 1.

In the above-mentioned (C'), "identity" is as defined for the above-mentioned (C).

The aptamer (II) of the present invention can also be (D) a conjugate of a plurality of one or more of the above-mentioned (A) and/or one or more of the above-mentioned (B) and/or one or more of the above-mentioned (C), preferably (D') a conjugate of a plurality of one or more of the above-mentioned (A') and/or one or more of the above-mentioned (B') and/or one or more of the above-mentioned (C'). In the above-mentioned (D), (D'), conjugation can be achieved by tandem binding. In the conjugation, a linker may be utilized. As the linker, nucleotide chains (e.g., 1 to about 20 nucleotides) and non-nucleotide chains (e.g., $-(CH_2)_n-$ linker, $-(CH_2CH_2O)_n-$linker, hexaethylene glycol linker, TEG linker, peptide-containing linker, -S-S- bond-containing linker, -CONH- bond-containing linker, $-OPO_3-$ bond-containing linker) can be mentioned. The plurality as mentioned in the above-described conjugate of a plurality thereof is not particularly limited, as long as it is two or more, and the plurality can be, for example, 2, 3 or 4.

Each nucleotide in the above-mentioned (A) - (D), (A') - (D') is the following (a) or (b):

(a)

(i) the 2'-position of the ribose of each pyrimidine nucleotide is a fluorine atom,
(ii) the 2'-position of the ribose of each purine nucleotide is a hydroxy group;

(b)

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

In addition, the aptamer (II) of the present invention contains, in the above-mentioned (B) or (C), a nucleotide sequence shown by the formula: GWAACAGGUYYYGCU wherein W is A or U, and Y is U or C) (SEQ ID NO: 49), and the nucleotide sequence can form a potential secondary structure shown by the formula (I'):

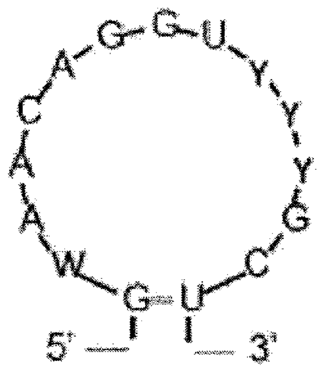

(I')

or the formula (II):

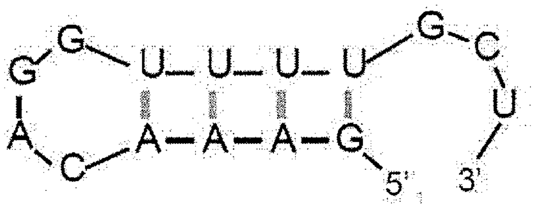

(II)

.

In the formula (I'), W is preferably A, and YYY is preferably UUU, UUC, UCU or CUU.

In addition, the aptamer (II) of the present invention can form a stem structure by an interaction between the sequences following the 5'-terminus and 3'-terminus of the nucleotide sequence represented by the formula: GWAACAGGUYYYGCU wherein W is A or U, and Y is U or C) (SEQ ID NO: 49) in the above-mentioned (B) or (C). While the stem length is not particularly limited, it is preferably not less than 4 base pairs. Also, the upper limit of the stem length is not particularly limited and, for example, it is not more than 18 base pairs, preferably not more than 11 base pairs. In the stem structure, even when base pairs are not formed in a part thereof, the aptamer activity is maintained as long as a

stem structure is constituted as a whole. Therefore, the stem structure part can contain substitution, deletion, insertion, addition and the like of nucleotides as long as the structure thereof is maintained.

The aptamer (II) of the present invention contains, in the above-mentioned (B') or (C'), a nucleotide sequence shown by the formula: GAAACAGGUUUUGCU (SEQ ID NO: 10) and the nucleotide sequence can form a potential secondary structure represented by the formula (I"):

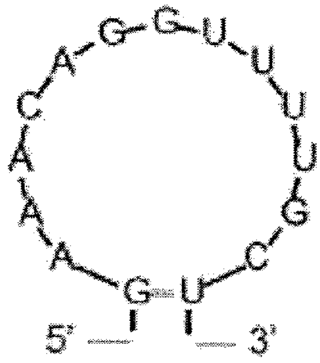


(I")

In the aptamer (II) of the present invention, a stem structure can be formed in the above-mentioned (B) or (C) by an interaction between the sequences following the 5'-terminus and 3'-terminus of the nucleotide sequence shown by the formula: GAAACAGGUUUUGCU (SEQ ID NO: 10). While the stem length is not particularly limited, it is preferably not less than 4 base pairs. While the upper limit of the stem length is not particularly limited, it is preferably not less than 10 base pairs. In the stem structure, even when base pairs are not formed in a part thereof, the aptamer activity is maintained as long as a stem structure is constituted as a whole. Therefore, the stem structure part can contain substitution, deletion, insertion, addition and the like of nucleotides as long as the structure thereof is maintained.

As mentioned above, in the aptamer of the present invention (encompassing the aptamers (I) and (II) of the present invention), at least one kind (e.g., 1, 2, 3 or 4 kinds) of nucleotide can also be a nucleotide comprising a hydroxyl group, or the above-described any atom or group, for example, at least two kinds (e.g., 2, 3 or 4 kinds) of atoms or groups selected from the group consisting of a fluorine atom, a hydroxyl group and a methoxy group, at the 2'-position of ribose.

In the aptamer of the present invention, all pyrimidine nucleotides may be a nucleotide wherein the 2'-position of ribose is a fluorine atom, or substituted by the aforementioned any atom or group, preferably, the same atom or group selected from the group consisting of a hydrogen atom, a hydroxyl group and a methoxy group.

In the aptamer of the present invention, all purine nucleotides may be a nucleotide wherein the 2'-position of ribose is a hydroxyl group, or substituted by the aforementioned any atom or group, preferably, the same atom or group selected from the group consisting of a hydrogen atom, a methoxy group and a fluorine atom.

The aptamer of the present invention can also be a nucleotide wherein all nucleotides have the aforementioned any atom or group, for example, the same atom or group selected from the group consisting of a hydrogen atom, a fluorine atom, a hydroxy group and a methoxy group, at the 2'-position of ribose.

In this Description, the nucleotides constituting the aptamer are assumed to be RNAs (i.e., the sugar groups are assumed to be ribose) in describing how the sugar groups are modified in the nucleotides. However, this does not mean that DNA is exempted from the aptamer-constituting nucleotides, and a modification should read as a modification of DNA as appropriate. When the nucleotide constituting the aptamer is DNA, for example, substitution of a hydroxyl group at the 2'-position of ribose by X should read as a substitution of one of the hydrogen atoms at the 2'-position of deoxyribose by X.

The aptamer of the present invention may be one wherein a sugar residue (e.g., ribose) of each nucleotide has been modified to increase the autotaxin binding activity, stability, drug deliverability and the like. As examples of the modification in a sugar residue, substitution of hydroxyl group at the 2'-position, the 3'-position and/or 4'-position of the sugar residue with another atom, and the like can be mentioned. As the kind of the modification, fluorination, alkoxylation (e.g., methoxylation, ethoxylation), O-arylation, S-alkylation (e.g., S-methylation, S-ethylation), S-arylation, and amination (e.g., $-NH_2$) can be mentioned. Such alterations in the sugar residue can be performed by a method known per se (see, for example, Sproat et al., (1991) Nucl. Acid. Res. 19, 733-738; Cotton et al., (1991) Nucl. Acid. Res. 19, 2629-2635; Hobbs et al., (1973) Biochemistry 12, 5138-5145). The sugar residue may also be BNA: Bridged nucleic acid (LNA: Linked nucleic acid), wherein a crosslinking structure is formed at the 2'-position and the 4'-position. Such alteration of the sugar residue can also be performed by a method known per se (e.g., Tetrahedron Lett., 38, 8735-8738 (1997); Tetrahedron, 59, 5123-5128 (2003), Rahman S.M.A., Seki S., Obika S., Yoshikawa H., Miyashita K., Imanishi T., J. Am. Chem. Soc., 130, 4886-4896 (2008) and the like).

The aptamer of the present invention may also have a nucleic acid base (e.g., purine or pyrimidine) altered (e.g., chemical substitution) to increase the autotaxin binding activity and the like. As examples of such alterations, pyrimidine alteration at 5-position, purine alteration at 6- and/or 8-position(s), alteration with an extracyclic amine, substitution with 4-thiouridine, and substitution with 5-bromo or 5-iodo-uracil can be mentioned.

In addition, the phosphate group contained in the aptamer of the present invention may be altered to confer resistance to nuclease, hydrolysis and increase the autotaxin binding activity. For example, the P(O)O group as a phosphoric acid group may be substituted by P(O)S (thioate), P(S)S (dithioate), P(O)$NR_2$ (amidate), P(O)R, R(O)OR', CO or $CH_2$ (formacetal) or 3'-amine (-NH-$CH_2$-$CH_2$-) [wherein each unit of R or R' is independently H or a substituted or unsubstituted alkyl (e.g., methyl, ethyl)].

Of these, one wherein at least one of the P(O)O groups to be the phosphoric acid group is substituted by P(O)S (thioate) or P(S)S (dithioate), i.e., phosphorothioated or phosphorodithioated, is preferable. When at least one of the phosphoric acid groups contained in the aptamer is phosphorothioated or phosphorodithioated, the activity of the aptamer of the present invention is improved. While the phosphoric acid group to be phosphorothioated or phosphorodithioated is not particularly limited, for example, it may be any nucleotide in the common sequence (SEQ ID NO: 10, 42 or 49) in the aptamer (I) or (II) of the present invention, and may be preferably a phosphoric acid group at the third A from the 5'-terminus of the common sequence.

The linking group is, for example, -O-, -N- or -S-, and nucleotides can bind to an adjoining nucleotide via these linking groups.

The alterations may also include alterations such as capping at 3' and 5'.

Terminal modification of the aptamer of the present invention can further be performed by adding, to the 5'-terminus and/or 3'-terminus of the aptamer, a polyethyleneglycol, amino acid, peptide, inverted dT, nucleic acid, nucleosides, myristoyl, lithocolic-oleyl, docosanyl, lauroyl, stearoyl, palmitoyl, oleoyl, linoleoyl, other lipids, steroids, cholesterol, caffeine, vitamins, dyes, fluorescent substances, anticancer agents, toxins, enzymes, radioactive substances, biotin and the like. As for such terminal modification, see, for example, US Patents 5,660,985 and 5,756,703.

In a particularly preferable embodiment, the present invention provides an aptamer that binds to an autotaxin and inhibits an activity thereof,

(1) which comprises a nucleotide sequence shown in SEQ ID NO: 16, wherein, in the nucleotide sequence, at least one phosphoric acid group (e.g., phosphoric acid group at the third A from 5'-terminus etc.) in a sequence shown by GAAACAGGUUUUGCU (SEQ ID NO: 10) is phosphorothioated or phosphorodithioated, and
(2) the 5'-terminus or 3'-terminus of the aptamer is modified by inverted dT or polyethylene glycol, respectively, and which is the following (a) or (b):

- (a) an aptamer wherein, in the nucleotides contained in the aptamer,
  - (i) the 2'-position of the ribose of each pyrimidine nucleotide is a fluorine atom,
  - (ii) the 2'-position of the ribose of each purine nucleotide is a hydroxy group;
- (b) the aptamer of (a), wherein
  - (i) the fluorine atom at the 2'-position of the ribose of each pyrimidine nucleotide is independently unsubstituted, or substituted by an atom or group selected from the group consisting of a hydrogen atom, a hydroxy group and a methoxy group,
  - (ii) the hydroxy group at the 2'-position of the ribose of each purine nucleotide is independently unsubstituted, or substituted by an atom or group selected from the group consisting of a hydrogen atom, a methoxy group and a fluorine atom.

As mentioned above, since the length of the aptamer of the present invention is preferably 23 - 50 nucleotides, or 23 - 40 nucleotides, the length of the above-mentioned aptamer containing a nucleotide sequence shown in SEQ ID NO: 16 (29 nucleotides) is preferably 29 - 50 nucleotide or 29 - 40 nucleotides. While the nucleotide sequences following the 5'-terminus and 3'-terminus of the nucleotide sequence shown in SEQ ID NO: 16 are not particularly limited as long as they can maintain the secondary structure that the nucleotide sequence shown in SEQ ID NO: 16 can take, it is desirable that the stem-loop structure of Fig. 1, right, can be further stabilized by extending the stem structure of Fig. 1, right (tolerating mismatch and formation of bulge).

The aptamer of the present invention can be synthesized as disclosed herein and by a method known per se in the art. One of the synthesis methods is a method using an RNA polymerase. The object RNA can be obtained by chemically synthesizing a DNA having the object sequence and a promoter sequence of RNA polymerase, followed by transcription using same as a template and according to an already-known method. It can be synthesized using DNA

polymerase. DNA having an object sequence is chemically synthesized and, using same as a template, amplification is performed by a known method of polymerase chain reaction (PCR). This is converted to a single strand by an already-known method of polyacrylamide electrophoresis or enzyme treatment method. When a modified aptamer is synthesized, the efficiency of elongation reaction can be increased by using a polymerase introduced with a mutation into a specific site. The thus-obtained aptamer can be purified easily by a known method.

Aptamer can be synthesized in a large amount by a chemical synthesis method such as amidite method, phosphoramidite method and the like. The synthesis method is a well-known method, and as described in Nucleic Acid (Vol. 2) [1] Synthesis and Analysis of Nucleic Acid (Editor: Yukio Sugiura, Hirokawa Publishing Company) and the like. In fact, a synthesizer such as OligoPilot100, OligoProcess and the like manufactured by GE Healthcare Bioscience is used. Purification is performed by a method known per se such as chromatography and the like.

A functional substance can be added to the aptamer after synthesis by introducing active groups such as amino group and the like during chemical synthesis by the phosphoramidite method and the like. For example, a polyethylene glycol chain introduced with a carboxyl group can be condensed by introducing an amino group into the terminal of the aptamer.

An aptamer binds to the target substance in a wide variety of binding modes, such as ionic bonds based on the negative charge of the phosphate group, hydrophobic bonds and hydrogen bonds based on ribose, and hydrogen bonds and stacking interaction based on nucleic acid bases. In particular, ionic bonds based on the negative charge of the phosphate group, which are present in the same number as the number of constituent nucleotides, are strong, and bind to lysine and arginine being present on the surface of the positive charge of protein. For this reason, nucleic acid bases not involved in the direct binding to the target substance can be substituted. In particular, because the region of stem structure has already formed base pairs and faces the inside of the double helical structure, nucleic acid bases are unlikely to bind directly to the target substance. Therefore, even when a base pair is substituted with another base pair, the activity of the aptamer often does not decrease. In structures wherein no base pairs are formed, such as loop structures, provided that the nucleic acid base is not involved in the direct binding to the target molecule, base substitution is possible. Regarding modifications of the 2'-position of ribose, the functional group at the 2'-position of ribose infrequently interacts directly with the target molecule, but in many cases, it is of no relevance, and can be substituted by another modified molecule. Hence, an aptamer, unless the functional group involved in the direct binding to the target molecule is substituted or deleted, often retains the activity thereof. It is also important that the overall three-dimensional structure does not change substantially.

An aptamer can be prepared by utilizing SELEX method and an improved method thereof (e.g., Ellington et al., (1990) Nature, 346, 818-822; Tuerk et al., (1990) Science, 249, 505-510). In the SELEX method, by setting strict selection conditions by increasing the number of rounds or using a competing substance, an aptamer exhibiting a stronger binding potential for the target substance is concentrated and selected. Hence, by adjusting the number of rounds of SELEX and/or changing the competitive condition, aptamers with different binding forces, aptamers with different binding modes, and aptamers with the same binding force or binding mode but different base sequences can be obtained in some cases. The SELEX method comprises a process of amplification by PCR; by causing a mutation by using manganese ions and the like in the process, it is possible to perform SELEX with higher diversity.

The aptamers obtained by SELEX are nucleic acids that exhibit high affinity for the target substance, but this does not mean inhibiting the physiological activity of the target substance. Autotaxin is a basic protein, and is considered to be likely to allow nucleic acids to bind thereto nonspecifically. An aptamer that does not bind to a specific site does not influence the activity of the target substance. In fact, the RNA containing a random sequence that was used as a negative control did not bind to or inhibit autotaxin.

Based on the active aptamer thus selected, SELEX can be performed using a different primer in an attempt to obtain an aptamer having a higher activity. As a specific method, SELEX is performed again after preparing a template wherein an aptamer with a determined sequence is partially randomized or a template doped with about 10 to 30% of random sequences.

An aptamer obtained by SELEX has a length of about 80 nucleotides, and this is difficult to prepare as a pharmaceutical as it is. Hence, it is necessary to repeat try-and-error efforts to shorten the aptamer to a length permitting easy chemical synthesis, which is 50 nucleotides or less. Depending on the primer design for an aptamer obtained by SELEX, the ease of the subsequent minimization operation changes. Unless the primer is designed successfully, subsequent development will be impossible even if an aptamer with activity is selected by SELEX. In the present invention, an aptamer maintaining an inhibitory activity could be obtained even with 23 nucleotides.

Aptamers are altered easily since they permit chemical synthesis. For aptamers, by predicting the secondary structure using the MFOLD program, or by predicting the steric structure by X-ray analysis or NMR analysis, it is possible to predict to some extent which nucleotide can be substituted or deleted, where to insert a new nucleotide and the like. A predicted aptamer with the new sequence can easily be chemically synthesized, and it can be determined whether or not the aptamer retains the activity using an existing assay system.

When a region important to the binding of the obtained aptamer with the target substance is identified by

repeated try-and-error efforts as described above, the activity remains unchanged in many cases even when a new sequence is added to both ends of the sequence. The length of the new sequence is not particularly limited.

Modifications, like sequences, permitting a wide range of design or alterations, can be freely performed by those of ordinary skill in the art.

As stated above, aptamers permit a wide range of design or alterations. The present invention also provides a production method of aptamer that enables a wide range of design or alteration of an aptamer comprising a specified sequence (e.g., a sequence corresponding to a portion selected from among stem regions, internal loop regions, bulge regions, hairpin loop regions and single-strand regions: hereinafter, abbreviated as fixed sequence as required).

For example, such production method of aptamer includes production of an aptamer comprising a fixed sequence by using a single kind of nucleic acid molecule consisting of a nucleotide sequence shown by:

sequence for primer (i) -(N)a-fixed sequence-(N)b- sequence for primer (ii)

wherein (N)a represents a nucleotide chain consisting of "a" units of N; (N)b represents a nucleotide chain consisting of "b" units of N; each of the units of N, whether identical or different, is a nucleotide selected from the group consisting of A, G, C, U and T (preferably, A, G, C and U). Each of "a" and "b", whether identical or different, can be any numbers, and can be, for example, 1 to about 100, preferably 1 to about 50, more preferably 1 to about 30, still more preferably 1 to about 20 or 1 to about 10], or plural kinds of nucleic acid molecules (e.g., library of nucleic acid molecule different in the number of a, b etc.) and primer pairs corresponding to the sequences for primer (i) and (ii), respectively.

The present invention also provides a complex comprising the aptamer of the present invention and a functional substance bound thereto. The binding between the aptamer and the functional substance in the complex of the present invention can be a covalent bond or a non-covalent bond. The complex of the present invention can be one wherein the aptamer of the present invention and one or more (e.g., 2 or 3) of functional substances of the same kind or different kinds are bound together. The functional substance is not particularly limited, as far as it newly confers a certain function to an aptamer of the present invention, or is capable of changing (e.g., improving) a certain characteristic which an aptamer of the present invention can possess. As examples of the functional substance, proteins, peptides, amino acids, lipids, sugars, monosaccharides, polynucleotides, and nucleotides can be mentioned. As examples of the functional substance, affinity substances (e.g., biotin, streptavidin, polynucleotides possessing affinity for target complementary sequence, antibodies, glutathione Sepharose, histidine), substances for labeling (e.g., fluorescent substances, luminescent substances, radioisotopes), enzymes (e.g., horseradish peroxidase, alkaline phosphatase), drug delivery vehicles (e.g., liposome, microspheres, peptides, polyethyleneglycols), drugs (e.g., those used in missile therapy such as calicheamycin and duocarmycin; nitrogen mustard analogues such as cyclophosphamide, melphalan, ifosfamide or trofosfamide; ethylenimines such as thiotepa; nitrosoureas such as carmustine; alkylating agents such as temozolomide or dacarbazine; folate-like metabolic antagonists such as methotrexate or raltitrexed; purine analogues such as thioguanine, cladribine or fludarabine; pyrimidine analogues such as fluorouracil, tegafur or gemcitabine; vinca alkaloids such as vinblastine, vincristine or vinorelbine and analogues thereof; podophyllotoxin derivatives such as etoposide, taxans, docetaxel or paclitaxel; anthracyclines such as doxorubicin, epirubicin, idarubicin and mitoxantrone, and analogues thereof; other cytotoxic antibiotics such as bleomycin and mitomycin; platinum compounds such as cisplatin, carboplatin and oxaliplatin; pentostatin, miltefosine, estramustine, topotecan, irinotecan and bicalutamide), and toxins (e.g., ricin toxin, liatoxin and vero toxin) can be mentioned. These functional molecules are finally removed in some cases. Furthermore, the molecules may be peptides that can be recognized and cleaved by enzymes such as thrombin, matrix metalloproteinase (MMP), and Factor X, and may be polynucleotides that can be cleaved by nucleases or restriction endonuclease.

The aptamer and the complex of the present invention can be used as, for example, a medicament, a diagnostic reagent, a test reagent or a reagent.

The aptamer and complex of the present invention can have an activity to inhibit the function of autotaxin. As mentioned above, autotaxin is deeply involved in the fibrosis of organ or tissue. Therefore, the aptamer and complex of the present invention is useful as a medicament for the treatment or prophylaxis of diseases involving organ or tissue fibrosis, particularly diseases associated with fibrosis of various tissues.

Here, examples of the diseases involving organ or tissue fibrosis include pulmonary fibrosis, prostatic hyperplasia, fibrosis of myocardium, myocardial fibrosis, musculoskeletal fibrosis, bone-marrow fibrosis, hysteromyoma, scleroderma, post-surgical adhesion, post-surgical scar, burn scar, hypertrophic scar, keloid, atopic dermatitis, peritoneal sclerosis, asthma, cirrhosis, chronic pancreatitis, scirrhous stomach cancer, hepatic fibrosis, renal fibrosis, fibrous vascular disease, retinopathy due to fibrous microvasculitis as complication of diabetes, neurosis, nephropathy, glomerulonephritis, renal tubule interstitial nephritis, hereditaryrenal diseases, arteriosclerosis peripheral arteritis and the like.

Autotaxin has an enzyme activity, and cleaves a physiologically active substance to be the substrate thereof. LPA is mainly produced from LPC, and LPA binds to a receptor thereof expressed on a cellular surface, activates intracellular G protein, and further, PLC, ERK and Rho at the downstream thereof, and exhibits physiological actions such as cell proliferation, survival, and migration. Therefore, the aptamer and complex of the present invention can be used as medicaments, diagnostic agents, test drugs, or reagents for diseases relating to activation of these pathways. As the disease, the above-mentioned diseases involving organ or tissue fibrosis can be mentioned.

The medicament of the present invention can be one formulated with a pharmaceutically acceptable carrier. As examples of the pharmaceutically acceptable carrier, excipients such as sucrose, starch, mannit, sorbit, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrants such as starch, carboxymethylcellulose, hydroxypropylstarch, sodium-glycol-starch, sodium hydrogen carbonate, calcium phosphate, and calcium citrate; lubricants such as magnesium stearate, Aerosil, talc, and sodium lauryl sulfate; flavoring agents such as citric acid, menthol, glycyrrhizin-ammonium salt, glycine, and orange powder; preservatives such as sodium benzoate, sodium hydrogen sulfite, methylparaben, and propylparaben; stabilizers such as citric acid, sodium citrate, and acetic acid; suspending agents such as methylcellulose, polyvinylpyrrolidone, and aluminum stearate; dispersing agents such as surfactants; diluents such as water, physiological saline, and orange juice; base waxes such as cacao butter, polyethylene glycol, and kerosene; and the like can be mentioned, but these are not limitative.

While the administration route of the medicament of the present invention is not particularly limited, for example, oral administration and parenteral administration can be mentioned. Preparations suitable for oral administration are a solution prepared by dissolving an effective amount of ligand in a diluent such as water, physiological saline, or orange juice; capsules, sachets or tablets comprising an effective amount of ligand in solid or granular form; a suspension prepared by suspending an effective amount of active ingredient in an appropriate dispersant; an emulsion prepared by dispersing and emulsifying a solution of an effective amount of active ingredient in an appropriate dispersant, and the like.

The medicament of the present invention can be coated by a method known per se for the purpose of taste masking, enteric dissolution, sustained release and the like as necessary. As examples of coating agents used for the coating, hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, Eudragit (manufactured by Rohm, Germany, methacrylic acid/acrylic acid copolymer), dyes (e.g., red iron oxide, titanium dioxide and the like) and the like are used. The medicament may be a rapid-release preparation or sustained-release preparation. Examples of the base of the sustained release include liposome, atelocollagen, gelatin, hydroxyapatite, PLGA and the like.

As preparations suitable for parenteral administration (e.g., intravenous administration, subcutaneous administration, intramuscular administration, topical administration, intraperitoneal administration, intranasal administration, pulmonary administration and the like), aqueous and non-aqueous isotonic sterile injectable liquids are available, which may comprise an antioxidant, a buffer solution, a bacteriostatic agent, an isotonizing agent and the like. Aqueous and non-aqueous sterile suspensions can also be mentioned, which may comprise a suspending agent, a solubilizer, a thickener, a stabilizer, an antiseptic and the like. The preparation can be included in a container such as an ampoule or a vial in a unit dosage volume or in several divided doses. An active ingredient and a pharmaceutically acceptable carrier can also be freeze-dried and stored in a state that may be dissolved or suspended in an appropriate sterile vehicle just before use. Sustained-release preparations are also suitable preparations. The sustained-release preparations include sustained release from carriers or containers embedded in the body, such as artificial bones, biodegradable or non-degradable sponges, bags, drug pumps, osmotic pressure pumps and the like. Devices for continuous or intermittent, systemic or topical delivery from outside the body are also included in the scope of sustained-release preparations. Biodegradable bases include liposome, cationic liposome, poly (lactic-co-glycolic) acid (PLGA), atelocollagen, gelatin, hydroxyapatite, polysaccharide sizofiran. In addition to liquid injections and sustained-release preparations, inhalants and ointments are also acceptable. In the case of an inhalant, an active ingredient in a freeze-dried state is micronized and administered by inhalation using an appropriate inhalation device. An inhalant can be formulated as appropriate with a conventionally used surfactant, oil, seasoning, cyclodextrin or derivative thereof and the like as required.

Here, as examples of the surfactant, oleic acid, lecithin, diethylene glycol dioleate, tetrahydroflufuryl oleate, ethyl oleate, isopropyl myristate, glyceryl trioleate, glyceryl monolaurate, glyceryl monooleate, glyceryl monostearate, glyceryl monolysinoate, cetyl alcohol, stearyl alcohol, polyethyleneglycol 400, cetylpyridinium chloride, sorbitan trioleate (trade name, Span 85), sorbitan monoleate (trade name, Span 80), sorbitan monolaurate (trade name, Span 20), polyoxyethylene hardened castor oil (trade name, HCO-60), polyoxyethylene (20) sorbitan monolaurate (trade name, Tween 20), polyoxyethylene (20) sorbitan monooleate (trade name, Tween 80), lecithin of natural resource origin (trade name, Epiclon), oleylpolyoxyethylene (2) ether (trade name, Brij 92), stearyl polyoxyethylene (2) ether (trade name, Brij 72), lauryl polyoxyethylene (4) ether (trade name, Brij 30), oleylpolyoxyethylene (2) ether (trade name, Genapol 0-020), block copolymer of oxyethylene and oxypropylene (trade name, Synperonic) and the like can be mentioned. As examples of the oil, corn oil, olive oil, cottonseed oil, sunflower oil and the like can be mentioned. In the case of an ointment, an

appropriate pharmaceutically acceptable base (yellow petrolatum, white petrolatum, paraffin, plastibase, silicone, white ointment, beeswax, lard, vegetable oils, hydrophilic ointment, hydrophilic petrolatum, purified lanolin, hydrolyzed lanolin, water-absorbing ointment, hydrophilic plastibase, macrogol ointment and the like) is blended with an active ingredient, and used as a preparation.

An inhalant can be produced according to a conventional method. Specifically, an inhalant can be produced by powdering or liquefying the above-described aptamer and complex of the present invention, blending it in an inhalation propellant and/or carrier, and filling them in an appropriate inhalation vessel. When the above-described aptamer and complex of the present invention is a powder, an ordinary mechanical powder inhalator can be used; in the case of a liquid, an inhalator such as a nebulizer can be used. Here, as the propellant, conventionally known one can be widely used; chlorofluorocarbon-series compounds such as chlorofluorocarbon-11, chlorofluorocarbon-12, chlorofluorocarbon-21, chlorofluorocarbon-22, chlorofluorocarbon-113, chlorofluorocarbon-114, chlorofluorocarbon-123, chlorofluorocarbon-142c, chlorofluorocarbon-134a, chlorofluorocarbon-227, chlorofluorocarbon-C318, and 1,1,1,2-tetrafluoroethane, hydrocarbons such as propane, isobutane, and n-butane, ethers such as diethyl ether, compressed gases such as nitrogen gas and carbon dioxide gas and the like can be mentioned.

As examples of the surfactant, oleic acid, lecithin, diethylene glycol dioleate, tetrahydroflufuryl oleate, ethyl oleate, isopropyl myristate, glyceryl trioleate, glyceryl monolaurate, glyceryl monooleate, glyceryl monostearate, glyceryl monolysinoate, cetyl alcohol, stearyl alcohol, polyethyleneglycol 400, cetylpyridinium chloride, sorbitan trioleate (trade name, Span 85), sorbitan monoleate (trade name, Span 80), sorbitan monolaurate (trade name, Span 20), polyoxyethylene hardened castor oil (trade name, HCO-60), polyoxyethylene (20) sorbitan monolaurate (trade name, Tween 20), polyoxyethylene (20) sorbitan monooleate (trade name, Tween 80), lecithin of natural resource origin (trade name, Epiclon), oleylpolyoxyethylene (2) ether (trade name, Brij 92), stearyl polyoxyethylene (2) ether (trade name, Brij 72), lauryl polyoxyethylene (4) ether (trade name, Brij 30), oleylpolyoxyethylene (2) ether (trade name, Genapol 0-020), block copolymer of oxyethylene and oxypropylene (trade name, Synperonic) and the like can be mentioned. As examples of the oil, corn oil, olive oil, cottonseed oil, sunflower oil and the like can be mentioned. In the case of an ointment, an appropriate pharmaceutically acceptable base (yellow petrolatum, white petrolatum, paraffin, plastibase, silicone, white ointment, beeswax, lard, vegetable oils, hydrophilic ointment, hydrophilic petrolatum, purified lanolin, hydrolyzed lanolin, water-absorbing ointment, hydrophilic plastibase, macrogol ointment and the like) is blended with the aptamer of the present invention as an active ingredient, and used as a preparation.

The dosage of the medicament of the present invention varies depending on the kind and activity of active ingredient, seriousness of disease, animal species being the subject of administration, drug tolerability of the subject of administration, body weight, age and the like, and the usual dosage, based on the amount of active ingredient per day for an adult, can be about 0.0001 to about 100 mg/kg, for example, about 0.0001 to about 10 mg/kg, preferably about 0.005 to about 1 mg/kg.

The aptamer and complex of the present invention can specifically bind to an autotaxin. Therefore, the aptamer and complex of the present invention is useful as a probe for autotaxin detection. The probe is useful for in vivo imaging, blood concentration measurement, tissue staining, ELISA and the like of autotaxin. In addition, the probe is useful as a diagnostic agent, test drug, reagent and the like for diseases involving autotaxin (fibrosis, disease accompanied by malignant tumor etc.).

Also, based on the specific binding to an autotaxin, the aptamer and complex of the present invention can be used as a ligand for autotaxin separation and purification.

In addition, the aptamer and complex of the present invention can be used as a drug delivery agent to a part where autotaxin is localized in vivo.

The present invention also provides a solid phase carrier having the aptamer and the complex of the present invention immobilized thereon. As examples of the solid phase carrier, a substrate, a resin, a plate (e.g., multiwell plate), a filter, a cartridge, a column, and a porous material can be mentioned. The substrate can be one used in DNA chips, protein chips and the like; for example, nickel-PTFE (polytetrafluoroethylene) substrates, glass substrates, apatite substrates, silicon substrates, alumina substrates and the like, and substrates prepared by coating these substrates with a polymer and the like can be mentioned. As examples of the resin, agarose particles, silica particles, a copolymer of acrylamide and N,N'-methylenebisacrylamide, polystyrene-crosslinked divinylbenzene particles, particles of dextran crosslinked with epichlorohydrin, cellulose fiber, crosslinked polymers of aryldextran and N,N'-methylenebisacrylamide, monodispersed synthetic polymers, monodispersed hydrophilic polymers, Sepharose, Toyopearl and the like can be mentioned, and also resins prepared by binding various functional groups to these resins were included. The solid phase carrier of the present invention can be useful in, for example, purifying, detecting and quantifying autotaxin.

The aptamer and the complex of the present invention can be immobilized onto a solid phase carrier by a method known per se. For example, a method that introduces an affinity substance (e.g., those described above) or a predetermined functional group into the aptamer or the complex of the present invention, and then immobilizes the aptamer and complex onto a solid phase carrier via the affinity substance or predetermined functional group can be mentioned. The present invention also provides such methods. The predetermined functional group can be a functional

group that can be subjected to a coupling reaction; for example, an amino group, a thiol group, a hydroxyl group, and a carboxyl group can be mentioned. The present invention also provides an aptamer having such a functional group introduced thereinto.

The present disclosure also provides a method of purifying and concentrating autotaxin. In particular, the present disclosure makes it possible to separate autotaxin from other family proteins. The method of purification and concentration of the present invention can comprise adsorbing autotaxin to the solid phase carrier of the present invention, and eluting the adsorbed autotaxin with an eluent. Adsorption of autotaxin to the solid phase carrier of the present invention can be achieved by a method known per se. For example, an autotaxin-containing sample (e.g., bacterial or cell culture or culture supernatant, blood) is introduced into the solid phase carrier of the present invention or a composition containing the same. Autotaxin can be eluted using an eluent such as a neutral solution. There is no limitation on the neutral eluent, which can have a pH of, for example, about 6 to about 9, preferably about 6.5 to about 8.5, and more preferably about 7 to about 8. The neutral solution can also comprise, for example, urea, chelating agent (e.g., EDTA), sodium salt (e.g., NaCl), a potassium salt (e.g., KCl), a magnesium salt (e.g., $MgCl_2$), a surfactant (e.g., Tween 20, Triton, NP40), and glycerin. The method of purification and concentration of the present invention can further comprise washing the solid phase carrier using a washing solution after autotaxin adsorption. Examples of the washing solution include those containing urea, a chelating agent (e.g., EDTA), Tris, an acid, an alkali, Transfer RNA, DNA, surfactants such as Tween 20, salts such as NaCl and the like. The method of purification and concentration of the present invention can still further comprise heating the solid phase carrier. This step enables the regeneration and sterilization of the solid phase carrier.

The present disclosure also provides a method of detecting and quantifying autotaxin. In particular, the present disclosure makes it possible to detect and quantify autotaxin separately from the proteins of other family proteins. The method of detection and quantitation of the present invention can comprise measuring autotaxin by utilizing the aptamer of the present invention (e.g., by the use of the complex and solid phase carrier of the present invention). The method of detecting and quantifying autotaxin can be performed in the same manner as an immunological method, except that the aptamer of the present invention is used in place of an antibody. Therefore, by using the aptamer of the present invention as a probe in place of an antibody, in the same manner as such methods as enzyme immunoassay (EIA) (e.g., direct competitive ELISA, indirect competitive ELISA, sandwich ELISA), radioimmunoassay (RIA), fluorescent immunoassay (FIA), Western blot method, immunohistochemical staining method, and cell sorting method, detection and quantitation can be performed. It can also be used as a molecule probe for PET and the like. These methods can be useful in, for example, measuring autotaxin contents in living organisms or biological samples, and in diagnosing a disease associated with autotaxin.

The present disclosure also provides an autotaxin inhibitor comprising the aptamer of the present invention. Since the aptamer of the present invention can bind to an autotaxin and inhibit its functions, the functions of the autotaxin can be inhibited by using an agent containing the aptamer.

An aptamer to be contained in the agent is not particularly limited as long as it is the aptamer of the present invention described in the present specification.

The present invention is explained in more detail in the following by referring to Examples, which are not to be construed as limitative.

[Examples]

Example 1: Production of RNA aptamer that specifically binds to autotaxin - 1

RNA aptamer that specifically binds to an autotaxin was produced by the SELEX method. SELEX was performed by reference to the method of Ellington et al. (Ellington and Szostak, Nature 346, 818-822, 1990) and the method of Tuerk et al. (Tuerk and Gold, Science 249, 505-510, 1990). As a target substance of SELEX, His-tagged β autotaxin (Recombinant Human, manufactured by R&D, hereinafter to be indicated as autotaxin) immobilized on TALON Metal Affinity Resin (manufactured by Clontech) as a carrier was used. A carrier on which an autotaxin is immobilized was obtained by utilizing the histidine requirement of cobalt in the carrier, and by mixing them and reacting them for about 1 hr at room temperature. The amount of immobilized autotaxin was confirmed by examining the autotaxin solution before immobilizing and the supernatant immediately after solid phasing by SDS-PAGE. As a result of SDS-PAGE, a band of autotaxin was not detected in the supernatant and it was confirmed that almost all of the autotaxin used was coupled. About 50 pmol of autotaxin was immobilized on about 5 μL of a resin.

RNA (30N) of the random sequence used in the first round was obtained by transcribing a chemically-synthesized DNA by using DuraScribe™T7 Transcription Kit (manufactured by Epicentre). In the RNA obtained by this method, the 2'-position of the ribose of the pyrimidine nucleotide is fluorinated. As a DNA template, a DNA with length of 73 nucleotides and having a primer sequence on both ends of a random sequence of 30 nucleotides shown below was used. The DNA template and primer were produced by chemical synthesis.

DNA template sequence: 5'-CGGATACGTCTCACTTCGTCNNNNNNNNNNNNNNNNNNNNNNNNNNNNNNCAGAGCGTTTAGA GTGCGGTCCC-3' (SEQ ID NO: 1)
Primer Fwd: 5'-TAATACGACTCACTATAGGGACCGCACTCTAAACGCTCTG-3' (SEQ ID NO: 2)
Primer Rev: 5'-CGGATACGTCTCACTTCGTC-3' (SEQ ID NO: 3)

In DNA template (SEQ ID NO: 1), N is any combination of nucleotides (A, G, C or T). Primer Fwd contains a promoter sequence of T7 RNA polymerase.

The RNA pool was added to a carrier having immobilized autotaxin, and the mixture was maintained at room temperature for 30 min, after which RNA that did not bind to autotaxin was removed by washing the resin with solution A. Solution A is a mixed solution of 145 mM sodium chloride, 5.4 mM potassium chloride, 1.8 mM calcium chloride, 0.8 mM magnesium chloride, 20 mM Tris (pH 7.6), and 0.05% Tween20. RNA bound to autotaxin was added with solution B as an eluent, heat treated at 95°C for 10 min and recovered from the supernatant. Solution B is a mixture of 6M guanidine hydrochloride and solution A. The recovered RNA was amplified by RT-PCR, transcribed by DuraScribe™ T7 Transcription Kit and used as a pool for the next round. With the foregoing as one round, a similar operation was repeated plural times. From the third round, solution C was used as an eluent. Solution C is a mixed solution of 250 mM imidazole and solution A. After the completion of SELEX, PCR product was cloned to pGEM-T Easy vector (manufactured by Promega), and used to transform Escherichia coli line DH5α (manufactured by Toyobo). Plasmid was extracted from a single colony, the base sequence of the clone was examined by a DNA sequencer (3130xl Genetic Analyzer, manufactured by ABI).

After 8 rounds of SELEX, the sequences of 46 clones were examined to find convergence in the sequences. From them were obtained 6 clones consisting of the nucleotide sequence shown in SEQ ID NO: 4. The secondary structure of the aptamer predicted using the MFOLD program (M. Zuker, Nucleic Acids Res. 31(13), 3406-3415, 2003) is shown in Fig. 1.

The nucleotide sequence of SEQ ID NO: 4 is shown below. Unless particularly indicated, each sequence shown in the Examples is in the 5' to 3' direction and, in each nucleotide, purine (A and G) is 2'-OH (natural RNA type) and pyrimidine (U and C) is 2'-fluoro-modified form.

SEQ ID NO: 4:

GGGACCGCACUCUAAACGCUCUGAGGGAAACAGGUUUUGCUCCUCGGAGCGUGGACGAAGUGA
GACGUAUCCG

The binding activity of a nucleic acid consisting of the nucleotide sequence shown in SEQ ID NO: 4 (hereinafter "nucleic acid consisting of a nucleotide sequence shown in SEQ ID NO: X (aptamer)" is sometimes to be abbreviated as "nucleic acid (aptamer) of SEQ ID NO: X") to autotaxin was evaluated by the surface plasmon resonance method. For the measurement, Biacore T100 manufactured by GE Healthcare was used. The SA chips with streptavidin immobilized thereon were used as the sensor chips. Binding thereto was about 1500 RU of 16 nucleotide Poly dT of which biotin was bound to the 5'-terminus. The nucleic acids used as a ligand were added with Poly A (16 nucleotides) to the 3'-terminus, and were immobilized on SA chips by T-A annealing. The nucleic acids (20 μL) were injected at a flow rate of 20 μL/min to immobilize about 1500 RU of the nucleic acids. An autotaxin for analyte was prepared at 0.02 μM, and 20 μL thereof was injected. As a running buffer, solution A was used.

The measurement results are shown in Fig. 2 and Table 1. As a result of the measurement, it was found that the nucleic acid of SEQ ID NO: 4 binds to autotaxin. The nucleic acid pool (30N) which was used for the first round and contained a 30-nucleotide random sequence used as a negative control, showed a binding amount of not more than 10% of the nucleic acid of SEQ ID NO: 4, and was found to not bind thereto (indicated as "-"). The binding amount here shows the maximum Resonance Unit (RU) value.

Example 2: Production of RNA aptamer that specifically binds to autotaxin - 2

Using a random sequence of 40 nucleotides and having a primer sequence different from that in Example 1 as a template, SELEX was performed in the same manner as in Example 1. As a target substance of SELEX, His-tagged autotaxin (Recombinant Human, manufactured by R&D) immobilized on TALON Metal Affinity Resin (manufactured by Clontech) as a carrier was used. The sequences of the templates and primers used are shown below. The DNA template and primers were produced by chemical synthesis.

DNA template sequence: 5'-

GTACGAAGACGCATCTCACNNNNNNNNNNNNNNNNNNNNNNNNNNNNNNNNNNNNNNNNGTTC CATGTCCTGTTGCCACCC-3' (SEQ ID NO: 5)
Primer Fwd: 5'-TAATACGACTCACTATAGGGTGGCAACAGGACATGGAAC-3' (SEQ ID NO: 6)
Primer Rev: 5'-GTACGAAGACGCATCTCAC-3' (SEQ ID NO: 7)

N in the DNA template (SEQ ID NO: 5) is any combination of deoxyribonucleotides (A, G, C or T). Primer Fwd contains a promoter sequence of T7 RNA polymerase.

After 8 rounds of SELEX, the sequences of 48 clones were examined to find convergence in the sequences. Of these, 5 clones (sequences thereof are shown in SEQ ID NO: 8) including the common sequence shown in SEQ ID NO: 10, which are contained in the nucleotide sequence shown in SEQ ID NO: 4, were obtained. In addition, one clone of the nucleic acid of SEQ ID NO: 9 having a common sequence different in one base was obtained.

Each of the above-mentioned nucleotide sequences is shown below. The underline shows a common sequence (SEQ ID NO: 10) part, and [U] shows nucleotide different from the common sequence. Each sequence is shown in the 5' to 3' direction and, in each nucleotide, purine (A and G) is 2'-OH (natural RNA type), and pyrimidine (U and C) is 2'-fluoro-modified form.

SEQ ID NO: 8:
GGGUGGCAACAGGACAUGGAACGCGCCCCAACUGCUUGAAACAGGUUUUGCUGAGCAGUGAUG
UGAGAUGCGUCUUCGUAC

SEQ ID NO: 9:
GGGUGGCAACAGGACAUGGAACGCGCCCCAACUGCUUGAAAC[U]GGUUUUGCUGAGCAGUGA
UGUGAGAUGCGUCUUCGUAC

SEQ ID NO: 10:
GAAACAGGUUUUGCU

Whether the nucleic acids of SEQ ID NOs: 8 and 9 bind to autotaxin was evaluated by the surface plasmon resonance method. For the measurement, Biacore T100 manufactured by GE Healthcare was used, and the measurement was performed by the method shown below. About 2700 RU autotaxin was immobilized on a sensorchip surface of CM4 chip by using an amino coupling kit. The flow rate was 20 μL/min, and nucleic acids (20 μL) prepared to 0.3 μM were injected as an analyte. As a running buffer, solution A was used. The results are shown in Table 1. In Table 1, nucleic acids showing a binding amount of not more than 10% of that of the aptamer of SEQ ID NO: 4 were considered not binding and marked with (-), and ones not less than that were considered binding and marked with (+). The binding amount here shows the maximum Resonance Unit (RU) value. As a result of the measurement, it was found that the nucleic acid of SEQ ID NO: 8 binds to autotaxin. In addition, it was found that the nucleic acid pool (40N) used in the first round and containing a random sequence with 40 nucleotides, which was used as a negative control, does not bind. Furthermore, the nucleic acid of SEQ ID NO: 9 having one different base does not bind.

Example 3: Production of RNA aptamer that specifically binds to autotaxin - 3

Using a template having a random sequence with 30 nucleotides and a primer sequence different from the one used in Example 1, SELEX was performed in the same manner as in Example 1. As a target substance of SELEX, His-tagged autotaxin (Recombinant Human, manufactured by R&D) immobilized on TALON Metal Affinity Resin (manufactured by Clontech) as a carrier was used. The sequences of the templates and primers used are shown below. The DNA template and primers were produced by chemical synthesis. As the nucleotide for transcription, ribonucleotide (A and G and C) and deoxyribonucleotide (T) were used.

DNA template sequence: 5'-AAGCTTCGTAAGTCGCAGTCNNNNNNNNNNNNNNNNNNNNNNNNNNNNNNCAGCGTAGTAGTG GTCCTAACCC-3' (SEQ ID NO: 11)
Primer Fwd: 5'-TAATACGACTCACTATAGGGTTAGGACCACTACTACGCTG-3' (SEQ ID NO: 12)
Primer Rev: 5'-AAGCTTCGTAAGTCGCAGTC-3' (SEQ ID NO: 13)

N in the DNA template (SEQ ID NO: 11) is any combination of nucleotides (A, G, C or T). In addition, primer Fwd contains a promoter sequence of T7 RNA polymerase.

After 8 rounds of SELEX, the sequences of 52 clones were examined to find convergence in the sequences. However, a sequence having the common sequence shown in SEQ ID NO: 10 was not included.

Example 4: Measurement of autotaxin inhibitory activity - 1

Whether the nucleic acids of SEQ ID NOs: 4, 8, 9 inhibit the activity of autotaxin was evaluated by the following method. As a substrate of autotaxin, phosphodiester bond-containing synthetic substrate p-nitrophenyl thymidine 5'-monophosphate (pNP-TMP) (SIGMA) was selected (hereinafter to be referred to as NPP2 inhibitory assay). A phosphodiester bond is cleaved by hydrolysis, and p-nitrophenol is liberated. The p-nitrophenol develops a yellow color, and the color is detected. For the assay, a 96-well plate (96-Well EIA/RIA Polystyrene Plates, Costar) was used, and the amount of the reaction mixture was 200 μL. As the reaction mixture, solution A was used. Nucleic acids were prepared in solution A (100 μL), pNP-TMP (20 μL) adjusted to 10 mM in the reaction mixture A was added, and the mixture was stirred well and heated at 37°C for 5 min. On the other hand, 6 ng of autotaxin diluted with solution A was prepared (80 μL), and heated at 37°C for 5 min. After heating, they were mixed to start enzyme reaction. The final autotaxin concentration in the reaction solution was 0.3 nM, and the final substrate concentration was 1 mM. A plate containing the reaction mixture was heated at 37°C for 24 hr, placed in a microplate reader SpectraMax190 (manufactured by Molecular Devices) and the absorbance was determined at wavelength 405 nm. The absorbance when nucleic acids are not added as 100% (A0), an inhibitory rate was determined from the absorbance (A) of each test substance and according to the following formula.

$$\text{Enzyme activity rate} = (A/A0) \times 100$$

The concentration ($IC_{50}$) of an inhibitor necessary for inhibiting the enzyme activity by 50% was determined. When 30N or 40N nucleic acid pool was used as a control (negative control), and when a known autotaxin inhibitor S32836 (SIGMA) was used as a control (positive control), a similar treatment was also performed, and the measurement was conducted. The results thereof are shown in Table 1.

Table 1

| Binding activity to autotaxin and NPP2 inhibitory activity ($IC_{50}$ value) | | |
|---|---|---|
| | Binding activity by Biacore | NPP2 inhibitory assay $IC_{50}$ value (μM) |
| SEQ ID NO: 4 | + | 0.0070±0.0010 |
| SEQ ID NO: 8 | + | 0.049±0.021 |
| SEQ ID NO: 9 | - | >1.0 |
| 30N | - | >1.0 |
| 40N | - | >1.0 |
| S32826 | not measured | 0.015±0.011 |

In the Table, "-" shows a binding amount of not more than 10% of the aptamer shown in SEQ ID NO: 4, and "+" shows not less than that. The binding amount here means the maximum Resonance Unit (RU) value (same in the following Biacorebond test).

The $IC_{50}$ value shows mean of 2 - 3 measurements±standard deviation, and ">1.0" indicates that an inhibitory activity was not found in the concentration range up to 1.0 μM (same in the following NPP2 inhibitory assay).

As a result of the measurement, it was shown that the aptamers of SEQ ID NOs: 4 and 8 have an inhibitory activity against the phosphodiesterase activity of autotaxin. On the other hand, the aptamer of SEQ ID NO: 9 did not show both the binding activity by Biacore, and inhibitory activity, it was shown that the 6th A from the 5'-terminus of common sequence shown in SEQ ID NO: 10 cannot be substituted by U. In addition, 30N or 40N as a negative control did not show an inhibitory activity ($IC_{50}$>1-0 μM). The $IC_{50}$ value of S32836 as a positive control was 0.015 μM.

Example 5: Chain shortening of aptamer

Aptamers of SEQ ID NOs: 4, 8 were subjected to chain shortening. The sequences of the altered forms are

shown in SEQ ID NOs: 14 - 24. Of the obtained aptamers, the secondary structure prediction of the aptamer shown in SEQ ID NO: 16 is shown in Fig. 1, right.

Each nucleotide sequence is shown below. The underline shows a common sequence (SEQ ID NO: 10) part, and [] shows a substituted nucleotide. Each sequence is shown in the 5' to 3' direction and, in each nucleotide, purine (A and G) is 2'-OH (natural RNA type), and pyrimidine (U and C) is 2'-fluoro-modified form.

SEQ ID NO: 14:(sequence of aptamer shown in SEQ ID NO: 4, after chain shortening to length of 46 nucleotides including the common sequence, wherein GGG is added to 5'-terminus and CCC is added to 3'-terminus)
GGGCUAAACGCUCUGAGGGAAACAGGUUUUGCUCCUCGGAGCGUGGACGCCC
SEQ ID NO: 15:(sequence of aptamer shown in SEQ ID NO: 4, after chain shortening to length of 37 nucleotides including the common sequence, wherein A at 5'-terminus is substituted by C and U at 3'-terminus by G)
[C]CGCUCUGAGGGAAACAGGUUUUGCUCCUCGGAGCG[G]
SEQ ID NO: 16:(sequence of aptamer shown in SEQ ID NO: 4, after chain shortening to length of 29 nucleotides including the common sequence)
UCUGAGGGAAACAGGUUUUGCUCCUCGGA
SEQ ID NO: 17:(sequence after chain shortening of aptamer shown in SEQ ID NO: 4 to length of 25 nucleotides including the common sequence)
UGAGGGAAACAGGUUUUGCUCCUCG
SEQ ID NO: 18:(sequence after chain shortening of aptamer shown in SEQ ID NO: 4 to length of 27 nucleotides including the common sequence)
CUGAGGGAAACAGGUUUUGCUCCUCGG
SEQ ID NO: 19:(sequence of aptamer shown in SEQ ID NO: 4, after chain shortening to length of 25 nucleotides including the common sequence, wherein one nucleotide is substituted)
[C]GAGGGAAACAGGUUUUGCUCCUCG
SEQ ID NO: 20:(sequence after chain shortening of aptamer shown in SEQ ID NO: 4 to length of 23 nucleotides including the common sequence)
GAGGGAAACAGGUUUUGCUCCUC
SEQ ID NO: 21:(sequence after chain shortening of aptamer shown in SEQ ID NO: 4 to length of 21 nucleotides including the common sequence)
AGGGAAACAGGUUUUGCUCCU
SEQ ID NO: 22:(sequence after chain shortening of aptamer shown in SEQ ID NO: 4 to length of 19 nucleotides including the common sequence)
GGGAAACAGGUUUUGCUCC
SEQ ID NO: 23:(sequence after chain shortening of aptamer shown in SEQ ID NO: 4 to length of 17 nucleotides including the common sequence)
GGAAACAGGUUUUGCUC
SEQ ID NO: 24:(sequence after chain shortening of aptamer shown in SEQ ID NO: 8 to length of 29 nucleotides including the common sequence)
ACUGCUUGAAACAGGUUUUGCUGAGCAGU

SEQ ID NO: 14 was obtained by using the chemically synthesized DNA sequence shown below as a template, and transcription using DuraScribe™T7 Transcription Kit.
DNA template sequence: 5'-GGGCGTCCACGCTCCGAGGAGCAAAACCTGTTTCCCTCAGAGCGTTTAGCCCTATAGTGAGTC GTATTA-3' (SEQ ID NO: 25)

All nucleic acids of SEQ ID NOs: 15 - 24 were produced by chemical synthesis. Whether these nucleic acids bind to autotaxin was evaluated by the surface plasmon resonance method similar to Example 2. The measurement results are shown in Table 2. In Table 2, nucleic acids showing a binding amount of not more than 10% of that of the aptamer of SEQ ID NO: 4 were considered not binding and marked with (-), and ones not less than that were considered binding and marked with (+). The binding amount here shows the maximum Resonance Unit (RU) value.

In addition, the autotaxin inhibitory activity was measured by NPP2 inhibitory assay, similar to Example 4. The $IC_{50}$ value thereof are shown in Table 2.

As a result of NPP2 inhibitory assay, the aptamers shown in SEQ ID NOs: 14 - 20 and 24 showed a strong inhibitory activity as evidenced by the $IC_{50}$ value of not more than 100 nM. These aptamers all contain the common sequence shown in SEQ ID NO: 10. The secondary structure of the aptamers of SEQ ID NOs: 14 - 20 is a stem-loop structure wherein the common sequence part is shown by the formula (I"):

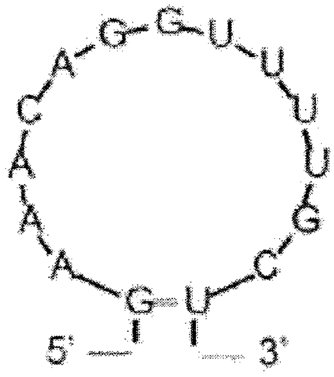

(I'')

,

and sequences following the 5'-terminus and 3'-terminus thereof form stems of various lengths. The aptamer of SEQ ID NO: 24 has a common sequence in which a part thereof is a stem structure. The aptamer consists of a common sequence part shown by the formula (II):

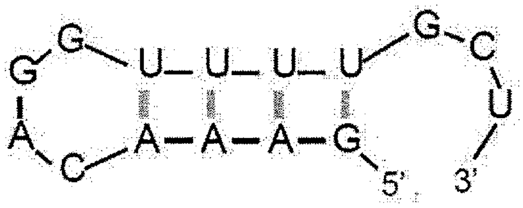

(II)

and a stem structure formed by sequences each following the 5'-terminus and the 3'-terminus thereof, and is close to the secondary structure of the aptamers of SEQ ID NOs: 14 - 20.

From the above, it was found that a sequence shown by SEQ ID NO: 10 is a sequence important for inhibiting autotaxin. From the results of the aptamer of SEQ ID NO: 20, it was found that a high inhibitory activity was maintained even after the sequence was short chained to 23 nucleotides.

Table 2

| Binding activity to autotaxin and NPP2 inhibitory activity ($IC_{50}$ value) | | | |
|---|---|---|---|
| SEQ ID NO: | Length | Binding activity by Biacore | NPP2 inhibitory assay $IC_{50}$ value (μM) |
| 14 | 52 | + | 0.026±0.0090 |
| 15 | 37 | + | 0.0070±0.0010 |
| 16 | 29 | + | 0.012±0.0020 |
| 17 | 25 | + | 0.037±0.0060 |
| 18 | 27 | + | 0.034±0.010 |
| 19 | 27 | + | 0.036±0.0040 |
| 20 | 23 | + | 0.10±0.0040 |
| 21 | 21 | + | 0.71±0.023 |
| 22 | 19 | - | 0.95±0.073 |
| 23 | 17 | - | >1.0 |
| 24 | 29 | + | 0.033±0.0020 |

Example 6: Effect of base substitution, deletion on short-chained aptamer

Based on the sequence shown in SEQ ID NO: 16, substitution, deletion of nucleotide was introduced, and an influence on the autotaxin inhibitory activity of the aptamer was examined. The sequences of the mutated aptamers

produced are shown in SEQ ID NOs: 26 - 37. The underline shows a common sequence (SEQ ID NO: 10) part, and [] shows a substituted nucleotide and "-" shows deletion. Each sequence is shown in the 5' to 3' direction and, in each nucleotide, purine (A and G) is 2'-OH (natural RNA type), and pyrimidine (U and C) is 2'-fluoro-modified form.

SEQ ID NO: 26:(sequence wherein 2nd A from 5'-terminus of common sequence shown in SEQ ID NO: 10 is substituted by U and 11th U by A)
UCUGAGGG[U]AACAGGUU[A]UGCUCCUCGGA
SEQ ID NO: 27:(sequence wherein 2nd A from 5'-terminus of common sequence shown in SEQ ID NO: 10 is substituted by U and 10th U by A)
UCUGAGGGA[U]ACAGGU[A]UUGCUCCUCGGA
SEQ ID NO: 28:(sequence wherein 4th A from 5'-terminus of common sequence shown in SEQ ID NO: 10 is substituted by U and 9th U by A)
UCUGAGGGAA[U]CAGG[A]UUUGCUCCUCGGA
SEQ ID NO: 29:(sequence wherein 3rd U from 5'-terminus of sequence shown in SEQ ID NO: 16 is substituted by C)
UC[C]GAGGGAAACAGGUUUUGCUCCUCGGA
SEQ ID NO: 30:(sequence wherein 3rd G from 3'-terminus of sequence shown in SEQ ID NO: 16 is substituted by A)
UCUGAGGGAAACAGGUUUUGCUCCUC [A]GA
SEQ ID NO: 31:(sequence wherein 9th U from 5'-terminus of common sequence shown in SEQ ID NO: 10 is substituted by C)
UCUGAGGGAAACAGG[C]UUUGCUCCUCGGA
SEQ ID NO: 32:(sequence wherein 12th U from 5'-terminus of common sequence shown in SEQ ID NO: 10 is substituted by C)
UCUGAGGGAAACAGGUUU[C]GCUCCUCGGA
SEQ ID NO: 33:(sequence wherein 2nd A from 5'-terminus of common sequence shown in SEQ ID NO: 10 is substituted by G)
UCUGAGGG[G]AACAGGUUUUGCUCCUCGGA
SEQ ID NO: 34:(sequence wherein 3rd A from 5'-terminus of common sequence shown in SEQ ID NO: 10 is substituted by G)
UCUGAGGGA[G]ACAGGUUUUGCUCCUCGGA
SEQ ID NO: 35:(sequence wherein 4th A from 5'-terminus of common sequence shown in SEQ ID NO: 10 is substituted by G)
UCUGAGGGAA[G]CAGGUUUUGCUCCUCGGA
SEQ ID NO: 36: (sequence wherein one base of 10th - 12th UUU from 5'-terminus of common sequence shown in SEQ ID NO: 10 is deleted)
UCUGAGGGAAACAGGUUU-GCUCCUCGGA
SEQ ID NO: 37: (sequence wherein one base of 2nd - 4th AAA from 5'-terminus of common sequence shown in SEQ ID NO: 10 is deleted)
UCUGAGGGAA-CAGGUUUUGCUCCUCGGA

All nucleic acids of SEQ ID NOs: 26 - 37 were produced by chemical synthesis. Whether these nucleic acids bind to autotaxin was evaluated by the surface plasmon resonance method similar to that in Example 2. The measurement results are shown in Table 3. As a result, it was found that the aptamers shown in SEQ ID NOs: 26, 29 - 34 bind to autotaxin.

Also, whether these nucleic acids show an autotaxin inhibitory activity was measured by NPP2 inhibitory assay similar to that in Example 4. The $IC_{50}$ values thereof are shown in Table 3. As a result, the aptamers of SEQ ID NOs: 29, 30, 32 showed a high inhibitory activity as evidenced by the $IC_{50}$ value of not more than 100 nM.

From the results of SEQ ID NO: 26 - 28 from the sequences included in Table 3, it was known that simultaneous introduction of mutation into the 2nd A and 11th U from the 5'-terminus of the common sequence shown in SEQ ID NO: 10 decreases inhibitory activity, and simultaneous introduction of mutation into the 3rd A and 10th U, or the 4th A and 9th U from the 5'-terminus of the common sequence more markedly decreases the inhibitory activity. While SEQ ID NOs: 29 and 30 showed a high inhibitory activity in Table 3, the secondary structures thereof predicted by the MFOLD program were found to be the same as that of SEQ ID NO: 16. They contain mutation in a stem part other than the common sequence part, and it was suggested that the activity is not influenced as long as the structure can be maintained even when some mutations are present in the stem part. On the other hand, it was found from the results of SEQ ID NO: 32 that the 12th U from the 5'-terminus contained in the common sequence could be substituted by C, even though the inhibitory activity decreased somewhat. However, from the results of SEQ ID NOs: 33 - 34, it was found that the second A from the 5'-terminus of the common sequence shown in SEQ ID NO: 10 cannot be converted to G, and substitution of the third A from the 5'-terminus with G decreases the inhibitory activity. From the results of SEQ ID NO: 35, it was found that substitution of the fourth A from the 5'-terminus of common sequence shown in SEQ ID NO: 10

with G markedly decreases both the binding activity and inhibitory activity, thus suggesting its importance for the binding and inhibitory activity. Also, from the results of SEQ ID NOs: 36 and 37, it was found that the lack of even one base of the common sequence markedly decreases the binding and inhibitory activity.

Table 3

| Binding activity of aptamer to autotaxin and NPP2 inhibitory activity ($IC_{50}$) | | |
|---|---|---|
| SEQ ID NO: | Binding activity by Biacore | NPP2 inhibitory assay $IC_{50}$ value (μM) |
| 16 | + | 0.012±0.0020 |
| 26 | + | 0.51±0.070 |
| 27 | - | >1.0 |
| 28 | - | >1.0 |
| 29 | + | 0.053±0.027 |
| 30 | + | 0.050±0.0010 |
| 31 | + | 0.13±0.046 |
| 32 | + | 0.055±0.006 |
| 33 | + | 0.69±0.28 |
| 34 | + | 0.34±0.020 |
| 35 | - | >1.0 |
| 36 | - | >1.0 |
| 37 | - | 0.72±0.39 |

Example 7: Production of autotaxin aptamer having higher activity - 1

SELEX was performed using an RNA pool wherein, of the sequence shown in SEQ ID NO: 16, 9% of random sequence was doped with the common sequence shown in SEQ ID NO: 10. SELEX was performed mostly similarly to Example 1. The DNA template and the primer sequence on the 5'-terminus side are shown below. As primer Rev, the nucleic acid of SEQ ID NO: 3 was used. The DNA template and primer were produced by chemical synthesis.
template

5'-
CGGATACGTCTCACTTCGTCCACGCTCCGAGGagcaaaacctgtttcCCTCAGAGCGTTTAGA
GTGCGGTCCC-3' (SEQ ID NO: 38)

a: a(91%), g(3%), c(3%), t(3%)
g: g(91%), a(3%), c(3%), t(3%)
c: c(91%), a(3%), g(3%), t(3%)
t: t(91%), a(3%), c(3%), g(3%)
primer Fwd: 5'-TAATACGACTCACTATAGGGACCGCACTCTAAACGC-3' (SEQ ID NO: 39)

After the completion of 2 rounds and 5 rounds, the sequences of 48 clones each (total 98 clones) were examined to find that almost all were the sequences shown in SEQ ID NO: 4. Of those, two were confirmed to have substituted nucleotides in the common sequence shown in SEQ ID NO: 10. They were short chained to a length of 29 nucleotides containing the common sequence part (SEQ ID NO: 40 and 41) and the sequences thereof are shown below. The underline shows a common sequence (SEQ ID NO: 10) part, and [] shows a substituted nucleotide and "-" shows deletion. Each sequence is shown in the 5' to 3' direction and, in each nucleotide, purine (A and G) is 2'-OH (natural RNA type), and pyrimidine (U and C) is 2'-fluoro-modified form. The underline shows a common sequence (SEQ ID NO: 10) part, and [] shows a substituted nucleotide. Each sequence is shown in the 5' to 3' direction and, in each nucleotide, purine (A and G) is 2'-OH (natural RNA type), and pyrimidine (U and C) is 2'-fluoro-modified form.

SEQ ID NO: 40:(sequence wherein 2nd A from 5'-terminus of common sequence shown in SEQ ID NO: 10 is substituted by U) UCUGAGGG[U]AACAGGUUUUGCUCCUCGGA
SEQ ID NO: 41:(sequence wherein 10th U from 5'-terminus of common sequence shown in SEQ ID NO: 10 is substituted by C) UCUGAGGGAAACAGGU[C]UUGCUCCUCGGA

All nucleic acids shown in SEQ ID NOs: 40 and 41 were produced by chemical synthesis. Whether these nucleic acids show an autotaxin inhibitory activity was measured by NPP2 inhibitory assay similar to that in Example 4. The $IC_{50}$ values thereof are shown in Table 4. As a result, all showed a high inhibitory activity as evidenced by the $IC_{50}$ value of not more than 100 nM against autotaxin. From the results of SEQ ID NO: 40, it was found that the second A from the 5'-terminus of the common sequence shown in SEQ ID NO: 10 can also be converted to U. Therefore, as the common sequence of the aptamer capable of binding to an autotaxin and inhibiting the activity thereof, GWAACAGGUUUUGCU (W is A or U) (SEQ ID NO: 42) was extracted. Also, from the results of SEQ ID NO: 41, it was found that the 10th U from the 5'-terminus of the common sequence shown in SEQ ID NO: 10 can be converted to C.
The aptamers of SEQ ID NOs: 40 and 41 both had a structure consisting of the common sequence (the above-mentioned formula (I)) and a stem structure. Particularly, SEQ ID NO: 41 had the same structure as SEQ ID NO: 16.

Example 8: Production of autotaxin aptamer having higher activity - 2

Furthermore, SELEX was performed using an RNA pool wherein, of the sequence shown in SEQ ID NO: 16, 9% of random sequence was doped with the sequence part other than the common sequence shown in SEQ ID NO: 10. SELEX was performed mostly similarly to Example 1. The template sequence is shown below. As the primer, the nucleic acid of SEQ ID NO: 39 and the nucleic acid of SEQ ID NO: 3 were used. The DNA template and primer were produced by chemical synthesis.
DNA template

5'-
CGGATACGTCTCACTTCGTCCACGCtccgaggAGCAAAACCTGTTTCcctcagaGCGTTTAGA
GTGCGGTCCC-3' (SEQ ID NO: 43)

a: a(91%), g(3%), c(3%), t(3%)
g: g(91%), a(3%), c(3%), t(3%)
c: c(91%), a(3%), g(3%), t(3%)
t: t(91%), a(3%), c(3%), g(3%)

After the completion of 5 rounds, the sequences of 48 clones each from 1, 3, 5 rounds (total 144 clones) were examined to find that almost all were the sequences shown in SEQ ID NO: 4. Of those, some were confirmed to have substituted bases in the sequence shown in SEQ ID NO: 16. They were short chained to a length of 29 nucleotides containing the common sequence (SEQ ID NO: 10) part (SEQ ID NOs: 44-48) and the sequences thereof are shown below. The underline shows a common sequence (SEQ ID NO: 10) part, and [] shows a substituted nucleotide and "-" shows deletion. Each sequence is shown in the 5' to 3' direction and, in each nucleotide, purine (A and G) is 2'-OH (natural RNA type), and pyrimidine (U and C) is 2'-fluoro-modified form. The underline shows a common sequence (SEQ ID NO: 10) part, and [] shows a substituted nucleotide. Each sequence is shown in the 5' to 3' direction and, in each nucleotide, purine (A and G) is 2'-OH (natural RNA type), and pyrimidine (U and C) is 2'-fluoro-modified form.
SEQ ID NO: 44:(sequence wherein 2 sites other than common sequence shown in SEQ ID NO: 10 are substituted) UCUG[G]GGGAAACAGGUUUUGCUCC[C]CGGA
SEQ ID NO: 45:(sequence wherein one site other than common sequence shown in SEQ ID NO: 10 is substituted) UCUGAGGGAAACAGGUUUUGCUCCUCG[A]A
SEQ ID NO: 46:(sequence wherein 11th U from 5'-terminus of common sequence shown in SEQ ID NO: 10 is substituted by C) UCUGAGGGAAACAGGUU[C]UGCUCCUCGGA
SEQ ID NO: 47:(sequence wherein 2 sites other than common sequence shown in SEQ ID NO: 10 are substituted) UC[C]GAGGGAAACAGGUUUUGCUCCUCG[C]A
SEQ ID NO: 48:(sequence wherein 2 sites other than common sequence shown in SEQ ID NO: 10 are substituted)

UCUGAG[U]GAAACAGGUUUGCU[G]CUCGGA

All nucleic acids shown in SEQ ID NOs: 44 - 48 were produced by chemical synthesis. Whether these nucleic acids show an autotaxin inhibitory activity was measured by a method similar to that in Example 4. The $IC_{50}$ values thereof are shown in Table 4. As a result, these aptamers were found to show a high inhibitory activity as evidenced by the $IC_{50}$ value of not more than 100 nM. From the results of SEQ ID NO: 46, the 11th U from the 5'-terminus of the common sequence (SEQ ID NO: 10 or 42) can be substituted by C. Combined with the results of the aforementioned SEQ ID NOs: 32 and 41, it was suggested that at least one U of the 10th - 12th UUU from the 5'-terminus of the common sequence can be substituted by C. Therefore, as the common sequence of the aptamer capable of binding to an autotaxin and inhibiting the activity thereof, GWAACAGGUYYYGCU (W is A or U, and Y is U or C) (SEQ ID NO: 49) was extracted.

Also, as regards the part other than the common sequence, it was shown that the activity is not influenced as long as the secondary structure consisting of the common sequence (the above-mentioned formula (I')) and a stem structure is maintained, even when the stem part is introduced with several mutations. Furthermore, from the results of SEQ ID NOs: 45 and 47 (two terminal nucleotides do not have a stem structure in secondary structure prediction by MFOLD), the length of the stem structure does not need to be 7, and may be shorter. This is consistent with the presence of activity in the aptamer of SEQ ID NO: 17.

Example 9: Production of autotaxin aptamer having higher activity - 3

Furthermore, SELEX was performed using an RNA pool wherein, of the sequence shown in SEQ ID NO: 16, 45% of random sequence was doped with the sequence part other than the common sequence shown in SEQ ID NO: 10. SELEX was performed mostly similarly to Example 1. As the DNA template, SEQ ID NO: 43 was used. As the primer, the nucleic acid of SEQ ID NO: 39 and the nucleic acid of SEQ ID NO: 3 were used.

a: a(55%), g(15%), c(15%), t(15%)
g: g(55%), a(15%), c(15%), t(15%)
c: c(55%), a(15%), g(15%), t(15%)
t: t(55%), a(15%), c(15%), g(15%)

After 6 rounds of SELEX, the sequences of 48 clones were examined to find no convergence; however, sequences wherein doped part was substituted by various bases were obtained. They were short chained to a length of 29 or 30 nucleotides containing the common sequence (SEQ ID NOs: 50-53) and the sequences thereof are shown below. The underline shows a common sequence (SEQ ID NO: 10) part, and [] shows a substituted nucleotide and "-" shows deletion. Each sequence is shown in the 5' to 3' direction and, in each nucleotide, purine (A and G) is 2'-OH (natural RNA type), and pyrimidine (U and C) is 2'-fluoro-modified form. The underline shows a common sequence (SEQ ID NO: 10) part, and [] shows a substituted nucleotide. Each sequence is shown in the 5' to 3' direction and, in each nucleotide, purine (A and G) is 2'-OH (natural RNA type), and pyrimidine (U and C) is 2'-fluoro-modified form.

SEQ ID NO: 50:(sequence with length of 30 nucleotides wherein 7 sites other than common sequence shown in SEQ ID NO: 10 are substituted)
U[A][G][A]GA[U]GGAAACAGGUUUUGCUC[A]UC[U][C]A
SEQ ID NO: 51:(sequence wherein 5 sites other than common sequence shown in SEQ ID NO: 10 are substituted)
U[U]UGA[A]GGAAACAGGUUUUGCUC[U]UCG[A][G]
SEQ ID NO: 52:(sequence wherein 4 sites other than common sequence shown in SEQ ID NO: 10 are substituted)
U[U][C]GAGGGAAACAGGUUUUGCUCCUC[A][A]A
SEQ ID NO: 53:(sequence wherein 7 sites other than common sequence shown in SEQ ID NO: 10 are substituted)
U[G][G]GA[A]GGAAACAGGUUUUGCUC[U]UC[A][C][C]

All nucleic acids shown in SEQ ID NOs: 50 - 53 were produced by chemical synthesis. Whether these nucleic acids show an autotaxin inhibitory activity was measured by a method similar to that in Example 4. The $IC_{50}$ values thereof are shown in Table 4. As a result, all showed an inhibitory activity against autotaxin. From the results of SEQ ID NOs: 50 - 53, it was suggested that bases at 4, 5 or 7 sites other than the common sequence shown in SEQ ID NO: 10 can be substituted.

[Table 4]

| Inhibitory activity ($IC_{50}$) of aptamer against autotaxin | |
|---|---|
| SEQ ID NO: | NPP2 inhibitory assay $IC_{50}$ (μM) |
| 40 | 0.049±0.0080 |
| 41 | 0.043±0.010 |
| 44 | 0.038±0.0010 |
| 45 | 0.029±0.0080 |
| 46 | 0.050±0.011 |
| 47 | 0.066±0.024 |
| 48 | 0.044±0.0030 |
| 50 | 0.021±0.0050 |
| 51 | 0.085±0.010 |
| 52 | 0.059±0.0020 |
| 53 | 0.030±0.0080 |

From the above results of Examples 6 - 9, it was known that the 2nd A from the 5'-terminus of common sequence shown in SEQ ID NO: 10 can be substituted by U, and at least one U of the 10th - 12th UUU from the 5'-terminus of the common sequence can be substituted by C. That is, as the common sequence of the aptamer capable of binding to an autotaxin and inhibiting the activity thereof, GWAACAGGUYYYGCU (W is A or U, and Y is U or C) (SEQ ID NO: 49) was extracted. It was also shown that the part other than the common sequence shown in SEQ ID NO: 10 can have 1 - 7 substitutions.

Example 10: Modification of chain-shortened aptamer

An altered form of the aptamer shown in SEQ ID NO: 16 having a modified terminus, and an altered form wherein modification has been introduced into the 2'-position of ribose of purine nucleotide in the sequence and altered forms including substitution by deoxyribonucleotide were produced. The sequences thereof are shown in SEQ ID NOs: 16(1) - 16(68). All nucleic acids were produced by chemical synthesis.

Each nucleotide sequence is shown below. In each sequence, the parenthesis on the right of each nucleotide shows modification at the 2'-position thereof, F shows fluorine atom, and M shows methoxy group. In each nucleotide, a small letter s on the right shows phosphorothioation of the phosphoric acid group of the nucleotide, and ss shows phosphorodithioation. Other small letters show deoxyribonucleotides. In the terminus modification, idT shows inverted-dT, Y shows ssH linker, 40P shows polyethylene glycol of SUNBRIGHT GL2-400GS2, 80P shows polyethylene glycol of SUNBRIGHT GL2-800GS2, 40PP shows polyethylene glycol of SUNBRIGHT GL2-400TS, 80PP shows polyethylene glycol of SUNBRIGHT GL2-800TS, 80PPP shows polyethylene glycol of SUNBRIGHT GL4-800GS2. The underline shows the common sequence (SEQ ID NO: 10) part.

SEQ ID NO: 16(1):(sequence of aptamer shown in SEQ ID NO: 16 with methoxy-modification introduced thereinto)

U(F)C(F)U(F)G(M)A(M)G(M)G(M)<u>GAAAC(F)AGGU(F)U(F)U(F)U(F)GC(F)U(F)</u>C(F)C(F)U(F)C(F)G(M)G(M)A(M)

SEQ ID NO: 16(2):(sequence of aptamer shown in SEQ ID NO: 16 with modification introduced thereinto)

U(F)C(F)U(F)G(M)A(M)G(M)G(M)<u>G(F)A(M)AA(M)C(F)A(M)GGU(F)U(F)U(F)U(F)G(M)C(F)U(F)</u>C(F)C(F)U(F)C(F)G(M)G(M)A(M)

SEQ ID NO: 16(3):(sequence of aptamer shown in SEQ ID NO: 16(1) with idT modification introduced into both terminals thereof)
idT-U(F)C(F)U(F)G(M)A(M)G(M)G(M)GAAAC(F)AGGU(F)U(F)U(F)U(F)GC(F)U(F )C(F)C(F)U(F)C(F)G(M)G(M)A(M)-idT
SEQ ID NO: 16(4):(sequence of aptamer shown in SEQ ID NO: 16(2) with idT modification introduced into both terminals thereof)
idT-U(F)C(F)U(F)G(M)A(M)G(M)G(M)G(F)A(M)AA(M)C(F)A(M)GGU(F)U(F)U(F) U(F)G(M)C(F)U(F)C(F)C(F)U(F)C(F)G(M)G(M)A(M)-idT
SEQ ID NO: 16(5):(sequence of aptamer shown in SEQ ID NO: 16(4), wherein one site is substituted by deoxyribonucleotide)
idT-U(F)C(F)U(F)G(M)A(M)G(M)G(M)G(F)A(M)AA(M)C(F)A(M)gGU(F)U(F)U(F) U(F)G(M)C(F)U(F)C(F)C(F)U(F)C(F)G(M)G(M)A(M)-idT
SEQ ID NO: 16(6):(sequence of aptamer shown in SEQ ID NO: 16(4), wherein 2 sites are substituted by deoxyribonucleotide)
idT-U(F)C(F)U(F)G(M)A(M)G(M)G(M)G(F)A(M)AA(M)C(F)A(M)ggU(F)U(F)U(F) U(F)G(M)C(F)U(F)C(F)C(F)U(F)C(F)G(M)G(M)A(M)-idT
SEQ ID NO: 16(7):(sequence of aptamer shown in SEQ ID NO: 16(4) with phosphorothioate introduced thereinto)
idT-U(F)C(F)U(F)G(M)A(M)G(M)G(M)G(F)A(M)AsA(M)C(F)A(M)GGU(F)U(F)U(F )U(F)G(M)C(F)U(F)C(F)C(F)U(F)C(F)G(M)G(M)A(M)-idT
SEQ ID NO: 16(8):(sequence of aptamer shown in SEQ ID NO: 16(4) with 40kDa polyethylene glycol introduced into 5'-terminus thereof)
40PP-Y-U(F)C(F)U(F)G(M)A(M)G(M)G(M)G(F)A(M)AA(M)C(F)A(M)GGU(F)U(F)U(F) U(F)G(M)C(F)U(F)C(F)C(F)U(F)C(F)G(M)G(M)A(M)-idT
SEQ ID NO: 16(9):(sequence of aptamer shown in SEQ ID NO: 16(4) with 80kDa polyethylene glycol introduced into 5'-terminus thereof)
80PP-Y-U(F)C(F)U(F)G(M)A(M)G(M)G(M)G(F)A(M)AA(M)C(F)A(M)GGU(F)U(F)U(F) U(F)G(M)C(F)U(F)C(F)C(F)U(F)C(F)G(M)G(M)A(M)-idT
SEQ ID NO: 16(10):(sequence of aptamer shown in SEQ ID NO: 16(4), wherein 3 sites are substituted by deoxyribonucleotide)
idT-tctG(M)A(M)G(M)G(M)G(F)A(M)AA(M)C(F)A(M)GGU(F)U(F)U(F)U(F)G(M)C (F)U(F)C(F)C(F)U(F)C(F)G(M)G(M)A(M)-idT
SEQ ID NO: 16(11):(sequence of aptamer shown in SEQ ID NO: 16(4), wherein 2 sites are substituted by deoxyribonucleotide)
idT-U(F)C(F)U(F)G(M)A(M)G(M)G(M)G(F)A(M)AA(M)C(F)A(M)GGU(F)U(F)U(F) U(F)G(M)C(F)U(F)C(F)C(F)tcG(M)G(M)A(M)-idT
SEQ ID NO: 16(12):(sequence of aptamer shown in SEQ ID NO: 16(4), wherein 2 sites are substituted by deoxyribonucleotide)
idT-U(F)C(F)U(F)G(M)A(M)G(M)G(M)G(F)A(M)AA(M)C(F)A(M)GGU(F)U(F)U(F) U(F)G(M)C(F)U(F)ccU(F)C(F)G(M)G(M)A(M)-idT
SEQ ID NO: 16(13):(sequence of aptamer shown in SEQ ID NO: 16(4), wherein one site is substituted by deoxyribonucleotide)
idT-U(F)C(F)U(F)G(M)A(M)G(M)G(M)G(F)A(M)AA(M)C(F)A(M)GGU(F)U(F)U(F) U(F)G(M)C(F)tC(F)C(F)U(F)C(F)G(M)G(M)A(M)-idT
SEQ ID NO: 16(14):(sequence of aptamer shown in SEQ ID NO: 16(4), wherein one site is substituted by deoxyribonucleotide)
idT-U(F)C(F)U(F)G(M)A(M)G(M)G(M)gA(M)AA(M)C(F)A(M)GGU(F)U(F)U(F)U(F )G(M)C(F)U(F)C(F)C(F)U(F)C(F)G(M)G(M)A(M)-idT
SEQ ID NO: 16(15):(sequence of aptamer shown in SEQ ID NO: 16(4), wherein one site is substituted by deoxyribonucleotide)
idT-U(F)C(F)U(F)G(M)A(M)G(M)G(M)G(F)A(M)AA(M)C(F)A(M)GGU(F)U(F)U(F) U(F)G(M)cU(F)C(F)C(F)U(F)C(F)G(M)G(M)A(M)-idT
SEQ ID NO: 16(16):(sequence of aptamer shown in SEQ ID NO: 16(4), wherein one site is substituted by deoxyribonucleotide)
idT-U(F)C(F)U(F)G(M)A(M)G(M)G(M)G(F)A(M)AA(M)C(F)A(M)GGU(F)U(F)U(F) tG(M)C(F)U(F)C(F)C(F)U(F)C(F)G(M)G(M)A(M)-idT

SEQ ID NO: 16(17):(sequence of aptamer shown in SEQ ID NO: 16(4), wherein one site is substituted by deoxyribonucleotide)

idT-U(F)C(F)U(F)G(M)A(M)G(M)G(M)G(F)A(M)AA(M)C(F)A(M)GGU(F)U(F)tU(F )G(M)C(F)U(F)C(F)C(F)U(F)C(F)G(M)G(M)A(M)-idT

SEQ ID NO: 16(18):(sequence of aptamer shown in SEQ ID NO: 16(4), wherein one site is substituted by deoxyribonucleotide)

idT-U(F)C(F)U(F)G(M)A(M)G(M)G(M)G(F)A(M)AA(M)C(F)A(M)GGU(F)tU(F)U(F )G(M)C(F)U(F)C(F)C(F)U(F)C(F)G(M)G(M)A(M)-idT

SEQ ID NO: 16(19):(sequence of aptamer shown in SEQ ID NO: 16(4), wherein one site is substituted by deoxyribonucleotide)

idT-U(F)C(F)U(F)G(M)A(M)G(M)G(M)G(F)A(M)AA(M)C(F)A(M)GGtU(F)U(F)U(F )G(M)C(F)U(F)C(F)C(F)U(F)C(F)G(M)G(M)A(M)-idT

SEQ ID NO: 16(20):(sequence of aptamer shown in SEQ ID NO: 16(4), wherein one site is substituted by deoxyribonucleotide)

idT-U(F)C(F)U(F)G(M)A(M)G(M)G(M)G(F)A(M)AA(M)cA(M)GGU(F)U(F)U(F)U(F )G(M)C(F)U(F)C(F)C(F)U(F)C(F)G(M)G(M)A(M)-idT

SEQ ID NO: 16(21):(sequence of aptamer shown in SEQ ID NO: 16(6), wherein one site is substituted by deoxyribonucleotide)

idT-U(F)C(F)U(F)G(M)A(M)G(M)G(M)gA(M)AA(M)C(F)A(M)ggU(F)U(F)U(F)U(F )G(M)C(F)U(F)C(F)C(F)U(F)C(F)G(M)G(M)A(M)-idT

SEQ ID NO: 16(22):(sequence of aptamer shown in SEQ ID NO: 16(21) with phosphorothioate introduced thereinto)

idT-U(F)C(F)U(F)G(M)A(M)G(M)G(M)gA(M)AsA(M)C(F)A(M)ggU(F)U(F)U(F)U( F)G(M)C(F)U(F)C(F)C(F)U(F)C(F)G(M)G(M)A(M)-idT

SEQ ID NO: 16(23):(sequence of aptamer shown in SEQ ID NO: 16(22) with 40kDa polyethylene glycol introduced instead of 5'-terminus idT)

40P-Y-U(F)C(F)U(F)G(M)A(M)G(M)G(M)gA(M)AsA(M)C(F)A(M)ggU(F)U(F)U(F)U( F)G(M)C(F)U(F)C(F)C(F)U(F)C(F)G(M)G(M)A(M)-idT

SEQ ID NO: 16(24):(sequence of aptamer shown in SEQ ID NO: 16(21) with 40kDa polyethylene glycol introduced instead of 5'-terminus idT)

40P-Y-U(F)C(F)U(F)G(M)A(M)G(M)G(M)gA(M)AA(M)C(F)A(M)ggU(F)U(F)U(F)U(F )G(M)C(F)U(F)C(F)C(F)U(F)C(F)G(M)G(M)A(M)-idT

SEQ ID NO: 16(25):(sequence of aptamer shown in SEQ ID NO: 16(6), wherein 4 sites are substituted by deoxyribonucleotide)

idT-tctG(M)A(M)G(M)G(M)gA(M)AA(M)C(F)A(M)ggU(F)U(F)U(F)U(F)G(M)C(F) U(F)C(F)C(F)U(F)C(F)G(M)G(M)A(M)-idT

SEQ ID NO: 16(26):(sequence of aptamer shown in SEQ ID NO: 16(6), wherein 2 sites are substituted by deoxyribonucleotide)

idT-U(F)C(F)U(F)G(M)A(M)G(M)G(M)gA(M)AA(M)C(F)A(M)ggU(F)U(F)U(F)U(F )G(M)C(F)U(F)C(F)C(F)U(F)cG(M)G(M)A(M)-idT

SEQ ID NO: 16(27):(sequence of aptamer shown in SEQ ID NO: 16(6), wherein 2 sites are substituted by deoxyribonucleotide)

idT-U(F)C(F)U(F)G(M)A(M)G(M)G(M)gA(M)AA(M)C(F)A(M)ggU(F)U(F)U(F)U(F )G(M)C(F)U(F)C(F)C(F)tC(F)G(M)G(M)A(M)-idT

SEQ ID NO: 16(28):(sequence of aptamer shown in SEQ ID NO: 16(6), wherein 2 sites are substituted by deoxyribonucleotide)

idT-U(F)C(F)U(F)G(M)A(M)G(M)G(M)gA(M)AA(M)C(F)A(M)ggU(F)U(F)U(F)U(F )G(M)C(F)U(F)C(F)cU(F)C(F)G(M)G(M)A(M)-idT

SEQ ID NO: 16(29):(sequence of aptamer shown in SEQ ID NO: 16(6), wherein 2 sites are substituted by deoxyribonucleotide)
idT-U(F)C(F)U(F)G(M)A(M)G(M)G(M)gA(M)AA(M)C(F)A(M)ggU(F)U(F)U(F)U(F )G(M)C(F)U(F)cC(F)U(F)C(F)G(M)G(M)A(M)-idT
SEQ ID NO: 16(30):(sequence of clone shown in SEQ ID NO: 16(21) with ssH linker introduced instead of 5'-terminus idT)
Y-U(F)C(F)U(F)G(M)A(M)G(M)G(M)gA(M)AA(M)C(F)A(M)ggU(F)U(F)U(F)U(F )G(M)C(F)U(F)C(F)C(F)U(F)C(F)G(M)G(M)A(M)-idT
SEQ ID NO: 16(31):(sequence of aptamer shown in SEQ ID NO: 16(22) with ssH linker introduced instead of 5'-terminus idT)
Y-U(F)C(F)U(F)G(M)A(M)G(M)G(M)gA(M)AsA(M)C(F)A(M)ggU(F)U(F)U(F)U( F)G(M)C(F)U(F)C(F)C(F)U(F)C(F)G(M)G(M)A(M)-idT
SEQ ID NO: 16(32):(sequence of aptamer shown in SEQ ID NO: 16(21), wherein six sites are substituted by deoxyribonucleotide)
idT-tctG(M)A(M)G(M)G(M)gA(M)AA(M)C(F)A(M)ggU(F)U(F)U(F)U(F)G(M)C(F) U(F)ccU(F)cG(M)G(M)A(M)-idT
SEQ ID NO: 16(33)(sequence of aptamer shown in SEQ ID NO: 16(21), wherein 3 sites are substituted by deoxyribonucleotide)
idT-U(F)C(F)U(F)G(M)A(M)G(M)G(M)gA(M)AA(M)C(F)A(M)ggU(F)U(F)U(F)U(F )G(M)C(F)U(F)ccU(F)cG(M)G(M)A(M)-idT
SEQ ID NO: 16(34)(sequence of aptamer shown in SEQ ID NO: 16(32) with 40kDa polyethylene glycol introduced instead of 5'-terminus idT)
40P-Y-tctG(M)A(M)G(M)G(M)gA(M)AA(M)C(F)A(M)ggU(F)U(F)U(F)U(F)G(M)C(F) U(F)ccU(F)cG(M)G(M)A(M)-idT
SEQ ID NO: 16(35)(sequence of aptamer shown in SEQ ID NO: 16(33) with 40kDa polyethylene glycol introduced instead of 5'-terminus idT)
40P-Y-U(F)C(F)U(F)G(M)A(M)G(M)G(M)gA(M)AA(M)C(F)A(M)ggU(F)U(F)U(F)U(F )G(M)C(F)U(F)ccU(F)cG(M)G(M)A(M)-idT
SEQ ID NO: 16(36):(sequence of aptamer shown in SEQ ID NO: 16(32) with phosphorothioate introduced thereinto)
idT-tctG(M)A(M)G(M)G(M)gA(M)AsA(M)C(F)A(M)ggU(F)U(F)U(F)U(F)G(M)C(F )U(F)ccU(F)cG(M)G(M)A(M)-idT
SEQ ID NO: 16(37):(sequence of aptamer shown in SEQ ID NO: 16(33) with phosphorothioate introduced thereinto)
idT-U(F)C(F)U(F)G(M)A(M)G(M)G(M)gA(M)AsA(M)C(F)A(M)ggU(F)U(F)U(F)U( F)G(M)C(F)U(F)ccU(F)cG(M)G(M)A(M)-idT
SEQ ID NO: 16(38):(sequence of aptamer shown in SEQ ID NO: 16(21), wherein 2 sites are substituted by deoxyribonucleotide)
idT-U(F)C(F)U(F)G(M)A(M)G(M)G(M)gA(M)AA(M)C(F)A(M)ggU(F)U(F)U(F)U(F )G(M)C(F)U(F)cC(F)U(F)cG(M)G(M)A(M)-idT
SEQ ID NO: 16(39):(sequence of aptamer shown in SEQ ID NO: 16(38) with 40kDa polyethylene glycol introduced instead of 5'-terminus idT)
40P-Y-U(F)C(F)U(F)G(M)A(M)G(M)G(M)gA(M)AA(M)C(F)A(M)ggU(F)U(F)U(F)U(F )G(M)C(F)U(F)cC(F)U(F)cG(M)G(M)A(M)-idT
SEQ ID NO: 16(40):(sequence of aptamer shown in SEQ ID NO: 16(21), wherein one site is substituted by deoxyribonucleotide)
idT-tC(F)U(F)G(M)A(M)G(M)G(M)gA(M)AA(M)C(F)A(M)ggU(F)U(F)U(F)U(F)G( M)C(F)U(F)C(F)C(F)U(F)C(F)G(M)G(M)A(M)-idT
SEQ ID NO: 16(41):(sequence of aptamer shown in SEQ ID NO: 16(21), wherein one site is substituted by deoxyribonucleotide)
idT-U(F)cU(F)G(M)A(M)G(M)G(M)gA(M)AA(M)C(F)A(M)ggU(F)U(F)U(F)U(F)G( M)C(F)U(F)C(F)C(F)U(F)C(F)G(M)G(M)A(M)-idT

SEQ ID NO: 16(42):(sequence of aptamer shown in SEQ ID NO: 16(21), wherein one site is substituted by deoxyribonucleotide)
idT-
U(F)C(F)tG(M)A(M)G(M)G(M)gA(M)AA(M)C(F)A(M)ggU(F)U(F)U(F)U(F)G( M)C(F)U(F)C(F)C(F)U(F)C(F)G(M)G(M)A(M)-idT
SEQ ID NO: 16(43)(sequence of aptamer shown in SEQ ID NO: 16(21) with 80kDa polyethylene glycol introduced instead of 5'-terminus idT)
80P-Y-
U(F)C(F)U(F)G(M)A(M)G(M)G(M)gA(M)AA(M)C(F)A(M)ggU(F)U(F)U(F)U(F )G(M)C(F)U(F)C(F)C(F)U(F)C(F)G(M)G(M)A(M)-idT
SEQ ID NO: 16(44)(sequence of aptamer shown in SEQ ID NO: 16(43) with phosphorothioate introduced thereinto)
80P-Y-
U(F)C(F)U(F)G(M)A(M)G(M)G(M)gA(M)AsA(M)C(F)A(M)ggU(F)U(F)U(F)U( F)G(M)C(F)U(F)C(F)C(F)U(F)C(F)G(M)G(M)A(M)-idT
SEQ ID NO: 16(45)(sequence of aptamer shown in SEQ ID NO: 16(21) with 80kDa polyethylene glycol introduced instead of 5'-terminus idT)
80PPP-Y-
U(F)C(F)U(F)G(M)A(M)G(M)G(M)gA(M)AA(M)C(F)A(M)ggU(F)U(F)U(F)U(F )G(M)C(F)U(F)C(F)C(F)U(F)C(F)G(M)G(M)A(M)-idT
SEQ ID NO: 16(46)(sequence of aptamer shown in SEQ ID NO: 16(45) with phosphorothioate introduced thereinto)
80PPP-Y-
U(F)C(F)U(F)G(M)A(M)G(M)G(M)gA(M)AsA(M)C(F)A(M)ggU(F)U(F)U(F)U( F)G(M)C(F)U(F)C(F)C(F)U(F)C(F)G(M)G(M)A(M)-idT
SEQ ID NO: 16(47):(sequence of aptamer shown in SEQ ID NO: 16(21), wherein 3 sites are substituted by deoxyribonucleotide)
idT-
tC(F)U(F)G(M)A(M)G(M)G(M)gA(M)AA(M)C(F)A(M)ggU(F)U(F)U(F)U(F)G( M)C(F)U(F)cC(F)U(F)cG(M)G(M)A(M)-idT
SEQ ID NO: 16(48):(sequence of aptamer shown in SEQ ID NO: 16(47) with 80kDa polyethylene glycol introduced instead of 5'-terminus idT)
80P-Y-
tC(F)U(F)G(M)A(M)G(M)G(M)gA(M)AA(M)C(F)A(M)ggU(F)U(F)U(F)U(F)G( M)C(F)U(F)cC(F)U(F)cG(M)G(M)A(M)-idT
SEQ ID NO: 16(49):(sequence of aptamer shown in SEQ ID NO: 16(48) with phosphorothioate introduced thereinto)
80P-Y-tC(F)U(F)G(M)A(M)G(M)G(M)gA(M)AsA(M)C(F)A(M)ggU(F)U(F)U(F)U(F)G (M)C(F)U(F)cC(F)U(F)cG(M)G(M)A(M)-idT
SEQ ID NO: 16(50):(sequence of aptamer shown in SEQ ID NO: 16(38) with 80kDa polyethylene glycol introduced instead of 5'-terminus idT)
80P-Y-
U(F)C(F)U(F)G(M)A(M)G(M)G(M)gA(M)AA(M)C(F)A(M)ggU(F)U(F)U(F)U(F )G(M)C(F)U(F)cC(F)U(F)cG(M)G(M)A(M)-idT
SEQ ID NO: 16(51):(sequence of aptamer shown in SEQ ID NO: 16(50) with phosphorothioate introduced thereinto)
80P-Y-
U(F)C(F)U(F)G(M)A(M)G(M)G(M)gA(M)AsA(M)C(F)A(M)ggU(F)U(F)U(F)U( F)G(M)C(F)U(F)cC(F)U(F)cG(M)G(M)A(M)-idT
SEQ ID NO: 16(52):(sequence of aptamer shown in SEQ ID NO: 16(40) with 80kDa polyethylene glycol introduced instead of 5'-terminus idT)
80P-Y-
tC(F)U(F)G(M)A(M)G(M)G(M)gA(M)AA(M)C(F)A(M)ggU(F)U(F)U(F)U(F)G( M)C(F)U(F)C(F)C(F)U(F)C(F)G(M)G(M)A(M)-idT
SEQ ID NO: 16(53):(sequence of aptamer shown in SEQ ID NO: 16(52) with phosphorothioate introduced thereinto)
80P-Y-tC(F)U(F)G(M)A(M)G(M)G(M)gA(M)AsA(M)C(F)A(M)ggU(F)U(F)U(F)U(F)G (M)C(F)U(F)C(F)C(F)U(F)C(F)G(M)G(M)A(M)-idT
SEQ ID NO: 16(54):(sequence of aptamer shown in SEQ ID NO: 16(36) with 80kDa polyethylene glycol introduced instead of 5'-terminus idT)
80P-Y-
tctG(M)A(M)G(M)G(M)gA(M)AsA(M)C(F)A(M)ggU(F)U(F)U(F)U(F)G(M)C(F )U(F)ccU(F)cG(M)G(M)A(M)-idT
SEQ ID NO: 16(55):(sequence of aptamer shown in SEQ ID NO: 16(47) with phosphorothioate introduced thereinto,

wherein two sites are further introduced with methoxy modification)
idT-tC(M)U(M)G(M)A(M)G(M)G(M)gA(M)AsA(M)C(F)A(M)ggU(F)U(F)U(F)U(F)G(M)C(F)U(F)cC(F)U(F)cG(M)G(M)A(M)-idT

SEQ ID NO: 16(56):(sequence of aptamer shown in SEQ ID NO: 16(47) with phosphorothioate introduced thereinto, wherein two sites are further introduced with methoxy modification)
idT-tC(F)U(F)G(M)A(M)G(M)G(M)gA(M)AsA(M)C(F)A(M)ggU(F)U(F)U(F)U(F)G(M)C(F)U(F)cC(M)U(M)cG(M)G(M)A(M)-idT

SEQ ID NO: 16(57):(sequence of aptamer shown in SEQ ID NO: 16(47) with phosphorothioate introduced thereinto, wherein one site is further introduced with methoxy modification)
idT-tC(F)U(F)G(M)A(M)G(M)G(M)gA(M)AsA(M)C(F)A(M)ggU(F)U(F)U(F)U(F)G(M)C(F)U(M)cC(F)U(F)cG(M)G(M)A(M)-idT

SEQ ID NO: 16(58):(sequence of aptamer shown in SEQ ID NO: 16(47) with phosphorothioate introduced thereinto, wherein one site is further introduced with methoxy modification)
idT-tC(F)U(F)G(M)A(M)G(M)G(M)gA(M)AsA(M)C(F)A(M)ggU(F)U(F)U(F)U(F)G(M)C(M)U(F)cC(F)U(F)cG(M)G(M)A(M)-idT

SEQ ID NO: 16(59):(sequence of aptamer shown in SEQ ID NO: 16(47) with phosphorothioate introduced thereinto, wherein one site is further introduced with methoxy modification)
idT-tC(F)U(F)G(M)A(M)G(M)G(M)gA(M)AsA(M)C(F)A(M)ggU(M)U(F)U(F)U(F)G(M)C(F)U(F)cC(F)U(F)cG(M)G(M)A(M)-idT

SEQ ID NO: 16(60):(sequence of aptamer shown in SEQ ID NO: 16(47) with phosphorothioate introduced thereinto, wherein one site is further introduced with methoxy modification)
idT-tC(F)U(F)G(M)A(M)G(M)G(M)gA(M)AsA(M)C(F)A(M)ggU(F)U(F)U(F)U(M)G(M)C(F)U(F)cC(F)U(F)cG(M)G(M)A(M)-idT

SEQ ID NO: 16(61):(sequence of aptamer shown in SEQ ID NO: 16(47) with phosphorothioate introduced thereinto, wherein one site is further introduced with methoxy modification)
idT-tC(F)U(F)G(M)A(M)G(M)G(M)gA(M)AsA(M)C(F)A(M)ggU(F)U(M)U(F)U(F)G(M)C(F)U(F)cC(F)U(F)cG(M)G(M)A(M)-idT

SEQ ID NO: 16(62):(sequence of aptamer shown in SEQ ID NO: 16(47) with phosphorothioate introduced thereinto, wherein one site is further introduced with methoxy modification)
idT-tC(F)U(F)G(M)A(M)G(M)G(M)gA(M)AsA(M)C(F)A(M)ggU(F)U(F)U(M)U(F)G(M)C(F)U(F)cC(F)U(F)cG(M)G(M)A(M)-idT

SEQ ID NO: 16(63):(sequence of aptamer shown in SEQ ID NO: 16(49) wherein 5'-terminus polyethylene glycol is changed from 80 kDa to 40 kDa)
40P-Y-tC(F)U(F)G(M)A(M)G(M)G(M)gA(M)AsA(M)C(F)A(M)ggU(F)U(F)U(F)U(F)G(M)C(F)U(F)cC(F)U(F)cG(M)G(M)A(M)-idT

SEQ ID NO: 16(64):(sequence of aptamer shown in SEQ ID NO: 16(49) wherein kind of polyethylene glycol at 5'-terminus is changed)
80PPP-Y-tC(F)U(F)G(M)A(M)G(M)G(M)gA(M)AsA(M)C(F)A(M)ggU(F)U(F)U(F)U(F)G(M)C(F)U(F)cC(F)U(F)cG(M)G(M)A(M)-idT

SEQ ID NO: 16(65):(sequence of aptamer shown in SEQ ID NO: 16(49) wherein 5 sites are introduced with methoxy modification)
80P-Y-tC(M)U(M)G(M)A(M)G(M)G(M)gA(M)AsA(M)C(F)A(M)ggU(F)U(F)U(F)U(F)G(M)C(F)U(M)cC(M)U(M)cG(M)G(M)A(M)-idT

SEQ ID NO: 16(66):(sequence of aptamer shown in SEQ ID NO: 16(65) wherein 5'-terminus polyethylene glycol is changed from 80 kDa to 40 kDa)
40P-Y-tC(M)U(M)G(M)A(M)G(M)G(M)gA(M)AsA(M)C(F)A(M)ggU(F)U(F)U(F)U(F)G(M)C(F)U(M)cC(M)U(M)cG(M)G(M)A(M)-idT

SEQ ID NO: 16(67):(sequence of aptamer shown in SEQ ID NO: 16(49) wherein 6 sites are introduced with methoxy modification)
80P-Y-tC(M)U(M)G(M)A(M)G(M)G(M)gA(M)AsA(M)C(F)A(M)ggU(F)U(F)U(F)U(F)G(M)C(M)U(M)cC(M)U(M)cG(M)G(M)A(M)-idT

SEQ ID NO: 16(68):(sequence of aptamer shown in SEQ ID NO: 16(47) with phosphorothioate introduced thereinto, wherein one site is further substituted by deoxyribonucleotide)
idT-tC(F)U(F)G(M)A(M)G(M)G(M)gA(M)assA(M)C(F)A(M)ggU(F)U(F)U(F)U(F)G(M)C(F)U(F)cC(F)U(F)cG(M)G(M)A(M)-idT

All nucleic acids of SEQ ID NOs: 16(1) - 16(68) were produced by chemical synthesis. Whether these nucleic acids show an autotaxin inhibitory activity was measured by NPP2 inhibitory assay similar to that in Example 4. The

$IC_{50}$ values thereof are shown in Table 5. The aptamers shown in SEQ ID NOs: 16(1) - 16(68) showed a high inhibitory activity in NPP2 inhibitory assay as evidenced by the $IC_{50}$ value of not more than 100 nM.

The aptamers shown in SEQ ID NOs: 16(1) - 16(68) are different in the modification of the part of the common sequence shown in SEQ ID NO: 10 and stem part, and all showed an inhibitory activity against autotaxin. This means that the modification at the 2'-position of ribose can be changed variously.

It was also known that the activity is improved by applying phosphorothioate and phosphorodithioate modifications.

[Table 5-1]

| Inhibitory activity ($IC_{50}$) of aptamer against autotaxin | |
|---|---|
| SEQ ID NO: | NPP2 inhibitory assay $IC_{50}$ (μM) |
| 16(1) | 0.017±0.0 |
| 16(2) | 0.022±0.0010 |
| 16(3) | 0.010±0.0040 |
| 16(4) | 0.017±0.0030 |
| 16(5) | 0.0067±0.0010 |
| 16(6) | 0.0059±0.0020 |
| 16(7) | 0.0073±0.0000 |
| 16(8) | 0.042±0.013 |
| 16(9) | 0.032±0.0010 |
| 16(10) | 0.021±0.0010 |
| 16(11) | 0.027±0.000 |
| 16(12) | 0.037±0.017 |
| 16(13) | 0.052±0.020 |
| 16(14) | 0.0063±0.000 |
| 16(15) | 0.077±0.000 |
| 16(16) | 0.045±0.0020 |
| 16(17) | 0.046±0.0020 |
| 16(18) | 0.029±0.000 |
| 16(19) | 0.051±0.0020 |
| 16(20) | 0.044±0.0030 |
| 16(21) | 0.0022±0.000 |
| 16(22) | 0.00050±0.000 |
| 16(23) | 0.0011±0.000 |
| 16(24) | 0.0074±0.000 |
| 16(25) | 0.0023±0.0020 |
| 16(26) | 0.0021±0.0010 |
| 16(27) | 0.0032±0.0010 |
| 16(28) | 0.0027±0.001 |
| 16(29) | 0.0033±0.002 |
| 16(30) | 0.0044±0.002 |

[Table 5-2]

| Inhibitory activity ($IC_{50}$) of aptamer against autotaxin (continued) | |
|---|---|
| SEQ ID NO: | NPP2 inhibitory assay IC50 (μM) |
| 16(31) | 0.00080±0.000 |
| 16(32) | 0.0014±0.001 |
| 16(33) | 0.00060±0.000 |
| 16(34) | 0.0042±0.001 |
| 16(35) | 0.003010.001 |
| 16(36) | 0.00030±0.000 |
| 16(37) | 0.00025±0.000 |
| 16(38) | 0.0013±0.000 |
| 16(39) | 0.0038±0.003 |
| 16(40) | 0.00090±0.000 |
| 16(41) | 0.0022±0.000 |
| 16(42) | 0.0010±0.001 |
| 16(43) | 0.0030±0.002 |
| 16(44) | 0.0028±0.000 |
| 16(45) | 0.013±0.001 |
| 16(46) | 0.0020±0.000 |
| 16(47) | 0.0024±0.001 |
| 16(48) | 0.0078±0.000 |
| 16(49) | 0.0016±0.000 |
| 16(50) | 0.012±0.001 |
| 16(51) | 0.0028±0.000 |
| 16(52) | 0.0094±0.000 |
| 16(53) | 0.0027±0.000 |
| 16(54) | 0.0023±0.001 |
| 16(55) | 0.00040±0.0000 |
| 16(56) | 0.00020±0.0000 |
| 16(57) | 0.00030±0.0000 |
| 16(58) | 0.00060±0.00010 |
| 16(59) | 0.016±0.0018 |
| 16(60) | 0.023±0.013 |
| 16(61) | 0.023±0.0036 |
| 16(62) | 0.023±0.0020 |
| 16(63) | 0.00080±0.00030 |
| 16(64) | 0.0013±0.0010 |
| 16(65) | 0.00030±0.0000 |
| 16(66) | 0.00070±0.00050 |

(continued)

| Inhibitory activity ($IC_{50}$) of aptamer against autotaxin (continued) | |
|---|---|
| SEQ ID NO: | NPP2 inhibitory assay IC50 (μM) |
| 16(67) | 0.00060±0.0000 |
| 16(68) | 0.0017±0.00020 |

Example 11: Confirmation of specificity of autotaxin aptamer

Whether the aptamer shown in SEQ ID NO: 16(23) has a binding activity to FGF2 (PeproTech) was confirmed by the surface plasmon resonance method. Autotaxin was immobilized on a flow cell 2 and FGF2 was immobilized on a flow cell 3 (about 1100 RU), and the aptamer was injected. The rest was performed similarly to Example 2. As a result of the measurement, it was found that the aptamer shown in SEQ ID NO: 16(23) does not bind to FGF2 (Fig. 3). This shows that the aptamer of the present invention specifically binds to autotaxin.

Example 12: Effect of autotaxin aptamer on pulmonary fibrosis

The aptamer shown in SEQ ID NO: 16(49), which was produced in Example 10, was intraperitoneally administered to bleomycin-induced pulmonary fibrosis model mice, and the effect thereof was verified.
ICR line SPF mice (10-week-old, male, Charles River Laboratories Japan, Inc.) were intratracheally administered with bleomycin (50 μL) prepared with PBS at 770 μg/mL under anesthesia. From the next day of bleomycin administration, autotaxin aptamer solution dissolved in PBS containing 1 mM magnesium chloride, or PBS containing 1 mM magnesium chloride alone (vehicle group) was intraperitoneally administered once per day at a single dose of 100 μL. The dose of aptamer was two doses of 1 and 3 mg/kg/day. A non-treated control group was also reared for the same test period. At 21 days from the bleomycin administration, the test was completed, the lungs were isolated, and the left lung was cryopreserved for hydroxyproline measurement. Furthermore, the right lung was fixed with 10% formalin solution, tissue section was prepared and subjected to Masson's trichrome staining. Hydroxyproline was measured using Hydroxyproline Colorimetric Assay kit of BioVision, Inc. An evaluation method of the clinical score of fibrosis was as follows. That is, no growth of collagen fiber on the whole observation surface region is 0, up to about 25% is 1, up to about 25% - 50% is 2, up to about 50% - 75% is 3, and not less than 75% is 4. Statistically significant difference was evaluated by T-test for testing the control group and the vehicle group, Dunnett-test for testing vehicle group and a medicament administration group under homoscedasticity, and Steel test under heteroscedasticity.
The results are shown in Figs. 4 and 5. All results are shown by mean of 6 - 7 mice±S.E. In the Figures, ** shows $p<0.01$. Fig. 4 shows the hydroxyproline amount per weight of the left lung. Suppression was found in an aptamer 3 mg/kg administration group relative to the vehicle group. The fibrosis score in Fig. 5 shows a significant ($p<0.01$) suppression in an aptamer 3 mg/kg administration group relative to the vehicle group.
From the above, it was suggested that the aptamer shown in SEQ ID NO: 16(49) is usable as a therapeutic drug for pulmonary fibrosis.

[Industrial Applicability]

The aptamer or complex of the present invention can be useful as a medicament, or a diagnostic agent or a reagent for diseases such as fibrosis. The aptamer and complex of the present invention can also be useful for the purification and concentration of autotaxin, labeling of autotaxin, as well as detection and quantification of autotaxin.
This application is based on a patent application No. 2014-090755 filed in Japan (filing date: April 24, 2014).

## Claims

1. An aptamer that binds to an autotaxin, which comprises a nucleotide sequence represented by the following formula GWAACAGGUUUUGCU (SEQ ID NO: 42)
wherein W is A or U (provided that uracil is optionally thymine), and which is the following (a) or (b):

   (a) an aptamer wherein, in the nucleotides contained in the aptamer,

      (i) the 2'-position of the ribose of each pyrimidine nucleotide is a fluorine atom,
      (ii) the 2'-position of the ribose of each purine nucleotide is a hydroxy group;

   (b) the aptamer of (a), wherein

      (i) the fluorine atom at the 2'-position of the ribose of each pyrimidine nucleotide is independently unsubstituted, or substituted by an atom or group selected from the group consisting of a hydrogen atom, a hydroxy group and a methoxy group,
      (ii) the hydroxy group at the 2'-position of the ribose of each purine nucleotide is independently unsubstituted, or substituted by an atom or group selected from the group consisting of a hydrogen atom, a methoxy group and a fluorine atom.

2. The aptamer according to claim 1, wherein W is A.

3. The aptamer according to claim 1 or 2, which comprises the nucleotide sequence shown in SEQ ID NO: 16.

4. The aptamer according to claim 1, which comprises the nucleotide sequence shown in SEQ ID NO: 10 wherein, in SEQ ID NO: 10, 1 - several nucleotides are further substituted, inserted or added, and which is the following (a) or (b):

   (a) an aptamer wherein, in the substituted, inserted or added nucleotides,

      (i) the 2'-position of the ribose of each pyrimidine nucleotide is a fluorine atom,
      (ii) the 2'-position of the ribose of each purine nucleotide is a hydroxy group;

   (b) the aptamer of (a), wherein

      (i) the fluorine atom at the 2'-position of the ribose of each pyrimidine nucleotide is independently unsubstituted, or substituted by an atom or group selected from the group consisting of a hydrogen atom, a hydroxy group and a methoxy group,
      (ii) the hydroxy group at the 2'-position of the ribose of each purine nucleotide is independently unsubstituted, or substituted by an atom or group selected from the group consisting of a hydrogen atom, a methoxy group and a fluorine atom.

5. The aptamer according to claim 1, which comprises a nucleotide sequence of any of the following (A), (B) and (C):

   (A) a nucleotide sequence selected from SEQ ID NOs: 4, 8, 14 - 20, 24, 29, 30, 32, 40, 44, 45, 47, 48 and 50 - 53 (provided that uracil is optionally thymine);
   (B) a nucleotide sequence selected from SEQ ID NOs: 4, 8, 14 - 20, 24, 29, 30, 40, 44, 45, 47, 48 and 50 - 53 (provided that uracil is optionally thymine), wherein 1 - several nucleotides are substituted, deleted, inserted or added nucleotide sequence;
   (C) a nucleotide sequence having identity of not less than 60% with a nucleotide sequence selected from SEQ ID NOs: 4, 8, 14 - 20, 24, 29, 30, 40, 44, 45, 47, 48 and 50 - 53 (provided that uracil is optionally thymine); which is the following (a) or (b):

      (a) an aptamer wherein, in the nucleotides contained in the aptamer,

         (i) the 2'-position of the ribose of each pyrimidine nucleotide is a fluorine atom,
         (ii) the 2'-position of the ribose of each purine nucleotide is a hydroxy group;

      (b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

**6.** The aptamer according to claim 1, which comprises a nucleotide sequence of any of the following (A'), (B') and (C'):

(A') a nucleotide sequence shown in SEQ ID NO: 16 (provided that uracil is optionally thymine);
(B') a nucleotide sequence shown in SEQ ID NO: 16 (provided that uracil is optionally thymine) (excluding a sequence shown by GAAACAGGUUUUGCU (SEQ ID NO: 10)), wherein 1 - 7 nucleotides are substituted, deleted, inserted or added;
(C') a nucleotide sequence having identity of not less than 70% with a nucleotide sequence shown in SEQ ID NO: 16 (provided that uracil is optionally thymine) (excluding a sequence shown by GAAACAGGUUUUGCU (SEQ ID NO: 10); which is the following (a) or (b):

(a) an aptamer wherein, in the nucleotides contained in the aptamer,

(i) the 2'-position of the ribose of each pyrimidine nucleotide is a fluorine atom,
(ii) the 2'-position of the ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,
(ii) the hydroxy group at the 2'-position of the ribose of each purine nucleotide is independently unsubstituted, or substituted by an atom or group selected from the group consisting of a hydrogen atom, a methoxy group and a fluorine atom.

**7.** The aptamer according to any one of claims 1 to 6, which has a base length of not less than 23.

**8.** The aptamer according to any one of claims 1 to 7, wherein at least one nucleotide is modified or alteration.

**9.** The aptamer according to claim 8, which is modified by inverted dT or polyethylene glycol.

**10.** The aptamer according to claim 9, wherein the inverted dT or polyethylene glycol binds to 5'-terminus or 3'-terminus of the aptamer.

**11.** The aptamer according to any one of claims 1 to 10, wherein at least one phosphate group contained in the aptamer is phosphorothioated or phosphorodithioated.

**12.** The aptamer according to claim 1, which comprises a nucleotide sequence shown in SEQ ID NO: 16, wherein, in the nucleotide sequence, at least one phosphoric acid group in a sequence shown by GAAACAGGUUUUGCU (SEQ ID NO: 10) is phosphorothioated or phosphorodithioated, and the 5'-terminus or 3'-terminus of the aptamer is modified by inverted dT or polyethylene glycol, respectively, and which is the following (a) or (b):

(a) an aptamer wherein, in the nucleotides contained in the aptamer,

(i) the 2'-position of the ribose of each pyrimidine nucleotide is a fluorine atom,
(ii) the 2'-position of the ribose of each purine nucleotide is a hydroxy group;

(b) the aptamer of (a), wherein

(i) the fluorine atom at the 2'-position of the ribose of each pyrimidine nucleotide is independently unsubstituted, or substituted by an atom or group selected from the group consisting of a hydrogen atom, a hydroxy group and a methoxy group,

(ii) the hydroxy group at the 2'-position of the ribose of each purine nucleotide is independently unsubstituted, or substituted by an atom or group selected from the group consisting of a hydrogen atom, a methoxy group and a fluorine atom.

**13.** A complex comprising the aptamer according to any one of claims 1 to 12 and a functional substance.

**14.** A medicament comprising the aptamer according to any one of claims 1 to 12, or the complex according to claim 13.

**15.** The aptamer according to any one of claims 1 to 12, or the complex according to claim 13, for use in the treatment or prophylaxis of diseases involving organ or tissue fibrosis.

**Patentansprüche**

**1.** Aptamer, das an ein Autotaxin bindet, eine Nucleotidsequenz, die durch die folgende Formel
GWAACAGGUUUUGCU (SEQ ID Nr. 42)
wiedergegeben wird, wobei W = A oder U ist (mit der Maßgabe, dass Uracil gegebenenfalls Thymin ist), umfasst und bei dem es sich um das folgende (a) oder (b) handelt:

(a) ein Aptamer, bei dem in den in dem Aptamer enthaltenen Nucleotiden

(i) auf der 2'-Position der Ribose jedes Pyrimidinnucleotids ein Fluoratom sitzt;
(ii) auf der 2'-Position der Ribose jedes Purinnucleotids eine Hydroxygruppe sitzt;

(b) das Aptamer von (a), bei dem

(i) das Fluoratom auf der 2'-Position der Ribose jedes Pyrimidinnucleotids unabhängig unsubstituiert oder durch ein Atom oder eine Gruppe substituiert ist, das oder die aus der Gruppe ausgewählt ist, die aus einem Wasserstoffatom, einer Hydroxygruppe und einer Methoxygruppe besteht;
(ii) die Hydroxygruppe auf der 2'-Position der Ribose jedes Purinnucleotids unabhängig unsubstituiert oder durch ein Atom oder eine Gruppe substituiert ist, das oder die aus der Gruppe ausgewählt ist, die aus einem Wasserstoffatom, einer Methoxygruppe und einem Fluoratom besteht.

**2.** Aptamer gemäß Anspruch 1, wobei W = A ist.

**3.** Aptamer gemäß Anspruch 1 oder 2, umfassend die Nucleotidsequenz, die in SEQ ID Nr. 16 gezeigt ist.

**4.** Aptamer gemäß Anspruch 1, das die in SEQ ID Nr. 10 gezeigte Nucleotidsequenz umfasst, wobei 1 oder mehrere Nucleotide in SEQ ID Nr. 10 weiterhin substituiert, inseriert oder addiert sind, und bei dem es sich um das folgende (a) oder (b) handelt:

(a) ein Aptamer, bei dem in den substituierten, inserierten oder addierten Nucleotiden

(i) auf der 2'-Position der Ribose jedes Pyrimidinnucleotids ein Fluoratom sitzt;
(ii) auf der 2'-Position der Ribose jedes Purinnucleotids eine Hydroxygruppe sitzt;

(b) das Aptamer von (a), bei dem

(i) das Fluoratom auf der 2'-Position der Ribose jedes Pyrimidinnucleotids unabhängig unsubstituiert oder durch ein Atom oder eine Gruppe substituiert ist, das oder die aus der Gruppe ausgewählt ist, die aus einem Wasserstoffatom, einer Hydroxygruppe und einer Methoxygruppe besteht;
(ii) die Hydroxygruppe auf der 2'-Position der Ribose jedes Purinnucleotids unabhängig unsubstituiert oder durch ein Atom oder eine Gruppe substituiert ist, das oder die aus der Gruppe ausgewählt ist, die aus einem Wasserstoffatom, einer Methoxygruppe und einem Fluoratom besteht.

**5.** Aptamer gemäß Anspruch 1, das eine Nucleotidsequenz gemäß einer der folgenden (A), (B) und (C) umfasst:

(A) eine Nucleotidsequenz, die aus SEQ ID Nr. 4, 8, 14-20, 24, 29, 30, 32, 40, 44, 45, 47, 48 und 50-53

ausgewählt ist (mit der Maßgabe, dass Uracil gegebenenfalls Thymin ist);
(B) eine Nucleotidsequenz, die aus SEQ ID Nr. 4, 8, 14-20, 24, 29, 30, 40, 44, 45, 47, 48 und 50-53 ausgewählt ist (mit der Maßgabe, dass Uracil gegebenenfalls Thymin ist), wobei es sich bei 1 oder mehreren Nucleotiden um eine substituierte, deletierte, inserierte oder addierte Nucleotidsequenz handelt;
(C) eine Nucleotidsequenz, die nicht weniger als 60% Identität mit einer Nucleotidsequenz aufweist, die aus SEQ ID Nr. 4, 8, 14-20, 24, 29, 30, 40, 44, 45, 47, 48 und 50-53 ausgewählt ist (mit der Maßgabe, dass Uracil gegebenenfalls Thymin ist), und bei dem es sich um das folgende (a) oder (b) handelt:

(a) ein Aptamer, bei dem in den in dem Aptamer enthaltenen Nucleotiden

(i) auf der 2'-Position der Ribose jedes Pyrimidinnucleotids ein Fluoratom sitzt;
(ii) auf der 2'-Position der Ribose jedes Purinnucleotids eine Hydroxygruppe sitzt;

(b) das Aptamer von (a), bei dem

(i) das Fluoratom auf der 2'-Position der Ribose jedes Pyrimidinnucleotids unabhängig unsubstituiert oder durch ein Atom oder eine Gruppe substituiert ist, das oder die aus der Gruppe ausgewählt ist, die aus einem Wasserstoffatom, einer Hydroxygruppe und einer Methoxygruppe besteht;
(ii) die Hydroxygruppe auf der 2'-Position der Ribose jedes Purinnucleotids unabhängig unsubstituiert oder durch ein Atom oder eine Gruppe substituiert ist, das oder die aus der Gruppe ausgewählt ist, die aus einem Wasserstoffatom, einer Methoxygruppe und einem Fluoratom besteht.

**6.** Aptamer gemäß Anspruch 1, das eine Nucleotidsequenz gemäß einem der folgenden (A'), (B') und (C') umfasst:

(A') eine Nucleotidsequenz, die in SEQ ID Nr. 16 gezeigt ist (mit der Maßgabe, dass Uracil gegebenenfalls Thymin ist);
(B') eine Nucleotidsequenz, die in SEQ ID Nr. 16 gezeigt ist (mit der Maßgabe, dass Uracil gegebenenfalls Thymin ist) (ausgenommen eine durch GAAACAGGUUUUGCU (SEQ ID Nr. 10) gezeigte Sequenz), wobei 1 bis 7 Nucleotide substituiert, deletiert, inseriert oder addiert sind;
(C') eine Nucleotidsequenz, die nicht weniger als 70% Identität mit einer Nucleotidsequenz aufweist, die in SEQ ID Nr. 16 gezeigt ist (mit der Maßgabe, dass Uracil gegebenenfalls Thymin ist) (ausgenommen eine durch GAAACAGGUUUUGCU (SEQ ID Nr. 10) gezeigte Sequenz), und bei dem es sich um das folgende (a) oder (b) handelt:

(a) ein Aptamer, bei dem in den in dem Aptamer enthaltenen Nucleotiden

(i) auf der 2'-Position der Ribose jedes Pyrimidinnucleotids ein Fluoratom sitzt;
(ii) auf der 2'-Position der Ribose jedes Purinnucleotids eine Hydroxygruppe sitzt;

(b) das Aptamer von (a), bei dem

(i) das Fluoratom auf der 2'-Position der Ribose jedes Pyrimidinnucleotids unabhängig unsubstituiert oder durch ein Atom oder eine Gruppe substituiert ist, das oder die aus der Gruppe ausgewählt ist, die aus einem Wasserstoffatom, einer Hydroxygruppe und einer Methoxygruppe besteht;
(ii) die Hydroxygruppe auf der 2'-Position der Ribose jedes Purinnucleotids unabhängig unsubstituiert oder durch ein Atom oder eine Gruppe substituiert ist, das oder die aus der Gruppe ausgewählt ist, die aus einem Wasserstoffatom, einer Methoxygruppe und einem Fluoratom besteht.

**7.** Aptamer gemäß einem der Ansprüche 1 bis 6, das eine Basenlänge von nicht weniger als 23 aufweist.

**8.** Aptamer gemäß einem der Ansprüche 1 bis 7, wobei wenigstens ein Nucleotid modifiziert oder verändert ist.

**9.** Aptamer gemäß Anspruch 8, das durch invertiertes dT oder Polyethylenglycol modifiziert ist.

**10.** Aptamer gemäß Anspruch 9, wobei das invertierte dT oder Polyethylenglycol an den 5'-Terminus oder 3'-Terminus des Aptamers bindet.

**11.** Aptamer gemäß einem der Ansprüche 1 bis 10, wobei wenigstens eine Phosphatgruppe, die in dem Aptamer

enthalten ist, phosphorothioatiert oder phosphorodithioatiert ist.

**12.** Aptamer gemäß Anspruch 1, das eine Nucleotidsequenz umfasst, die in SEQ ID Nr. 16 gezeigt ist, wobei in der Nucleotidsequenz wenigstens eine Phosphorsäuregruppe in einer durch GAAACAGGUUUUGCU (SEQ ID Nr. 10) gezeigten Sequenz phosphorothioatiert oder phosphorodithioatiert ist und der 5'-Terminus oder 3'-Terminus des Aptamers durch invertiertes dT bzw. Polyethylenglycol modifiziert ist, und bei dem es sich um das folgende (a) oder (b) handelt:

(a) ein Aptamer, bei dem in den in dem Aptamer enthaltenen Nucleotiden

(i) auf der 2'-Position der Ribose jedes Pyrimidinnucleotids ein Fluoratom sitzt;
(ii) auf der 2'-Position der Ribose jedes Purinnucleotids eine Hydroxygruppe sitzt;

(b) das Aptamer von (a), bei dem

(i) das Fluoratom auf der 2'-Position der Ribose jedes Pyrimidinnucleotids unabhängig unsubstituiert oder durch ein Atom oder eine Gruppe substituiert ist, das oder die aus der Gruppe ausgewählt ist, die aus einem Wasserstoffatom, einer Hydroxygruppe und einer Methoxygruppe besteht;
(ii) die Hydroxygruppe auf der 2'-Position der Ribose jedes Purinnucleotids unabhängig unsubstituiert oder durch ein Atom oder eine Gruppe substituiert ist, das oder die aus der Gruppe ausgewählt ist, die aus einem Wasserstoffatom, einer Methoxygruppe und einem Fluoratom besteht.

**13.** Komplex, umfassend das Aptamer gemäß einem der Ansprüche 1 bis 12 und eine funktionelle Substanz.

**14.** Medikament, umfassend das Aptamer gemäß einem der Ansprüche 1 bis 12 oder den Komplex gemäß Anspruch 13.

**15.** Aptamer gemäß einem der Ansprüche 1 bis 12 oder Komplex gemäß Anspruch 13 zur Verwendung bei der Behandlung oder Prophylaxe von Erkrankungen, die eine Organ- oder Gewebefibrose beinhalten.

**Revendications**

**1.** Aptamère qui se lie à une autotaxine, qui comprend une séquence nucléotidique représentée par la formule suivante GWAACAGGUUUUGCU (SEQ ID NO : 42)
dans laquelle W est A ou U (à condition qu'un uracile soit optionnellement une thymine), et qui est l'un de (a) ou (b) suivants :

(a) un aptamère dans lequel, dans les nucléotides contenus dans l'aptamère,

(i) la position 2' du ribose de chaque nucléotide pyrimidine est un atome de fluor,
(ii) la position 2' du ribose de chaque nucléotide purine est un groupement hydroxy ;

(b) l'aptamère de (a), dans lequel

(i) l'atome de fluor en position 2' du ribose de chaque nucléotide pyrimidine est indépendamment non substitué, ou substitué par un atome ou un groupement sélectionné dans le groupe consistant en un atome d'hydrogène, un groupement hydroxy et un groupement méthoxy,
(ii) le groupement hydroxy en position 2' du ribose de chaque nucléotide purine est indépendamment non substitué, ou substitué par un atome ou un groupement sélectionné dans le groupe consistant en un atome d'hydrogène, un groupement méthoxy et un atome de fluor.

**2.** Aptamère selon la revendication 1, dans lequel W est A.

**3.** Aptamère selon la revendication 1 ou 2, qui comprend la séquence nucléotidique présentée dans SEQ ID NO : 16.

**4.** Aptamère selon la revendication 1, qui comprend la séquence nucléotidique présentée dans SEQ ID NO : 10 dans laquelle, dans SEQ ID NO : 10, 1 à plusieurs nucléotides sont en outre substitués, insérés ou ajoutés, et qui est l'un de (a) ou (b) suivants :

(a) un aptamère dans lequel, dans les nucléotides substitués, insérés ou ajoutés,

(i) la position 2' du ribose de chaque nucléotide pyrimidine est un atome de fluor,
(ii) la position 2' du ribose de chaque nucléotide purine est un groupement hydroxy ;

(b) l'aptamère de (a), dans lequel

(i) l'atome de fluor en position 2' du ribose de chaque nucléotide pyrimidine est indépendamment non substitué, ou substitué par un atome ou un groupement sélectionné dans le groupe consistant en un atome d'hydrogène, un groupement hydroxy et un groupement méthoxy,
(ii) le groupement hydroxy en position 2' du ribose de chaque nucléotide purine est indépendamment non substitué, ou substitué par un atome ou un groupement sélectionné dans le groupe consistant en un atome d'hydrogène, un groupement méthoxy et un atome de fluor.

**5.** Aptamère selon la revendication 1, qui comprend une séquence nucléotidique selon l'un quelconque des (A), (B) et (C) suivants :

(A) une séquence nucléotidique sélectionnée parmi les SEQ ID NO : 4, 8, 14 à 20, 24, 29, 30, 32, 40, 44, 45, 47, 48 et 50 à 53 (à condition qu'un uracile soit optionnellement une thymine) ;
(B) une séquence nucléotidique sélectionnée parmi les SEQ ID NO : 4, 8, 14 à 20, 24, 29, 30, 40, 44, 45, 47, 48 et 50 à 53 (à condition qu'un uracile soit optionnellement une thymine), dans laquelle 1 à plusieurs nucléotides sont une séquence nucléotidique substituée, délétée, insérée ou ajoutée ;
(C) une séquence nucléotidique présentant une identité pas inférieure à 60 % avec une séquence nucléotidique sélectionnée parmi les SEQ ID NO : 4, 8, 14 à 20, 24, 29, 30, 40, 44, 45, 47, 48 et 50 à 53 (à condition qu'un uracile soit optionnellement une thymine) ; qui est l'un de (a) ou (b) suivants :

(a) un aptamère dans lequel, dans les nucléotides contenus dans l'aptamère,

(i) la position 2' du ribose de chaque nucléotide pyrimidine est un atome de fluor,
(ii) la position 2' du ribose de chaque nucléotide purine est un groupement hydroxy ;

(b) l'aptamère de (a), dans lequel

(i) l'atome de fluor en position 2' du ribose de chaque nucléotide pyrimidine est indépendamment non substitué, ou substitué par un atome ou un groupement sélectionné dans le groupe consistant en un atome d'hydrogène, un groupement hydroxy et un groupement méthoxy,
(ii) le groupement hydroxy en position 2' du ribose de chaque nucléotide purine est indépendamment non substitué, ou substitué par un atome ou un groupement sélectionné dans le groupe consistant en un atome d'hydrogène, un groupement méthoxy et un atome de fluor.

**6.** Aptamère selon la revendication 1, qui comprend une séquence nucléotidique selon l'un quelconque des (A'), (B') et (C') suivants :

(A') une séquence nucléotidique présentée dans SEQ ID NO : 16 (à condition qu'un uracile soit optionnellement une thymine) ;
(B') une séquence nucléotidique présentée dans SEQ ID NO : 16 (à condition qu'un uracile soit optionnellement une thymine) (à l'exclusion d'une séquence présentée par GAAACAGGUUUUGCU (SEQ ID NO : 10)), dans laquelle 1 à 7 nucléotides sont substitués, délétés, insérés, ou ajoutés ;
(C') une séquence nucléotidique ayant une identité pas inférieure à 70 % avec une séquence nucléotidique présentée dans SEQ ID NO : 16 (à condition qu'un uracile soit optionnellement une thymine) (à l'exclusion d'une séquence présentée dans GAAACAGGUUUUGCU (SEQ ID NO : 10)) ; qui est l'un de (a) ou (b) suivants :

(a) un aptamère dans lequel, dans les nucléotides contenus dans l'aptamère,

(i) la position 2' du ribose de chaque nucléotide pyrimidine est un atome de fluor,
(ii) la position 2' du ribose de chaque nucléotide purine est un groupement hydroxy ;

(b) l'aptamère de (a), dans lequel

(i) l’atome de fluor en position 2’ du ribose de chaque nucléotide pyrimidine est indépendamment non substitué, ou substitué par un atome ou un groupement sélectionné dans le groupe consistant en un atome d’hydrogène, un groupement hydroxy et un groupement méthoxy,
(ii) le groupement hydroxy en position 2’ du ribose de chaque nucléotide purine est indépendamment non substitué, ou substitué par un atome ou un groupement sélectionné dans le groupe consistant en un atome d’hydrogène, un groupement méthoxy et un atome de fluor.

**7.** Aptamère selon l’une quelconque des revendications 1 à 6, qui a une longueur pas inférieure à 23 bases.

**8.** Aptamère selon l’une quelconque des revendications 1 à 7, dans lequel au moins un nucléotide est modifié ou présente une altération.

**9.** Aptamère selon la revendication 8, qui est modifié par une dT inversée ou un polyéthylène glycol.

**10.** Aptamère selon la revendication 9, dans lequel la dT inversée ou le polyéthylène glycol se lie à l’extrémité 5’-terminale ou à l’extrémité 3’-terminale de l’aptamère.

**11.** Aptamère selon l’une quelconque des revendications 1 à 10, dans lequel au moins un groupement phosphate contenu dans l’aptamère est phosphorothioé ou phosphorodithioé.

**12.** Aptamère selon la revendication 1, qui comprend une séquence nucléotidique présentée dans SEQ ID NO : 16, dans laquelle, dans la séquence nucléotidique, au moins un groupement acide phosphorique dans une séquence présentée par GAAACAGGUUUUGCU (SEQ ID NO : 10) est phosphorothioé ou phosphorodithioé, et l’extrémité 5’-terminale ou l’extrémité 3’-terminale de l’aptamère est modifié par une dT inversée ou un polyéthylène glycol, respectivement, et qui est l’un de (a) ou (b) suivants :

(a) un aptamère dans lequel, dans les nucléotides contenus dans l’aptamère,

(i) la position 2’ du ribose de chaque nucléotide pyrimidine est un atome de fluor,
(ii) la position 2’ du ribose de chaque nucléotide purine est un groupement hydroxy ;

(b) l’aptamère de (a), dans lequel

(i) l’atome de fluor en position 2’ du ribose de chaque nucléotide pyrimidine est indépendamment non substitué, ou substitué par un atome ou un groupement sélectionné dans le groupe consistant en un atome d’hydrogène, un groupement hydroxy et un groupement méthoxy,
(ii) le groupement hydroxy en position 2’ du ribose de chaque nucléotide purine est indépendamment non substitué, ou substitué par un atome ou un groupement sélectionné dans le groupe consistant en un atome d’hydrogène, un groupement méthoxy et un atome de fluor.

**13.** Complexe comprenant l’aptamère selon l’une quelconque des revendications 1 à 12 et une substance fonctionnelle.

**14.** Médicament comprenant l’aptamère selon l’une quelconque des revendications 1 à 12, ou le complexe selon la revendication 13.

**15.** Aptamère selon l’une quelconque des revendications 1 à 12, ou le complexe selon la revendication 13, pour son utilisation dans le traitement ou la prophylaxie de maladies impliquant une fibrose d’un organe ou d’un tissu.

Fig. 1

SEQ ID NO: 4

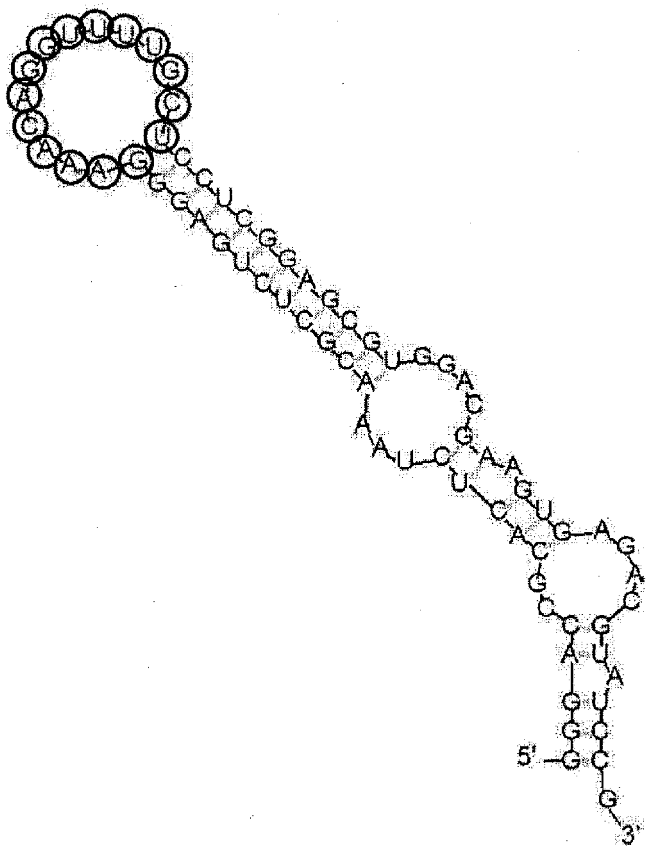


SEQ ID NO: 16

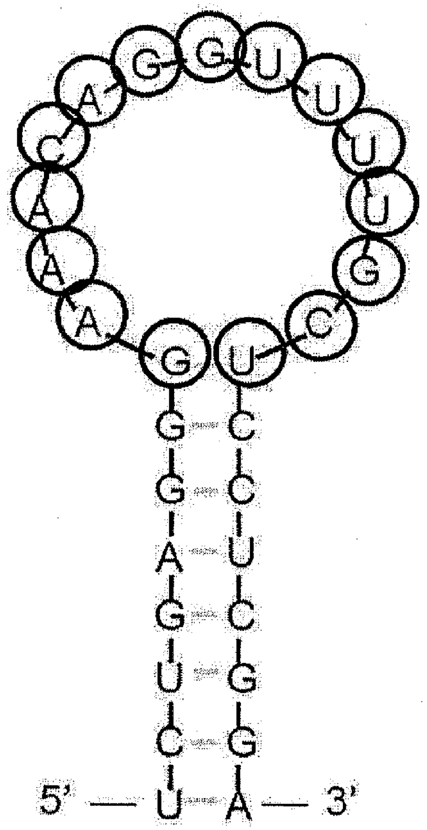

Fig. 2

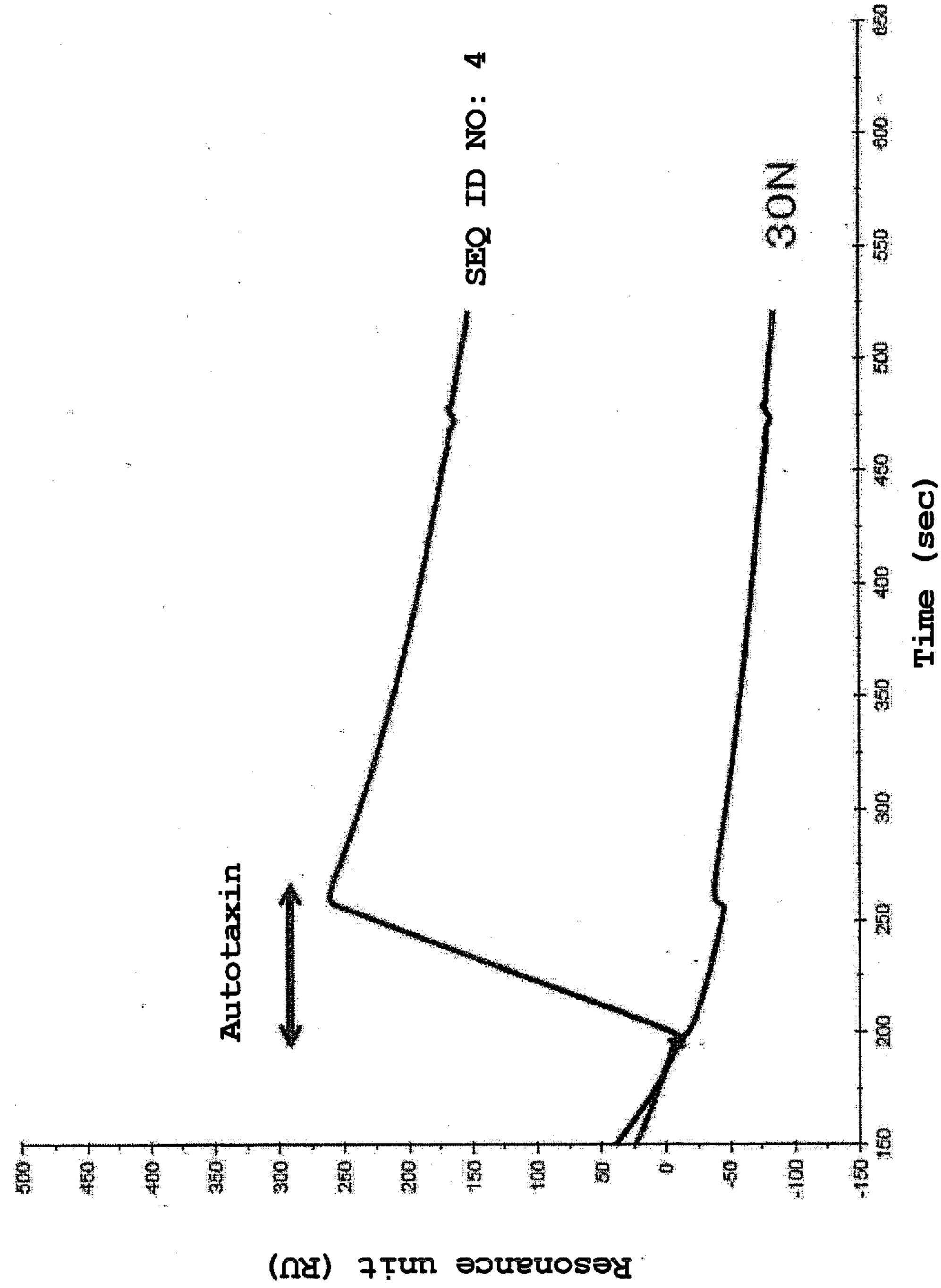
Autotaxin
SEQ ID NO: 4
30N
Time (sec)
Resonance unit (RU)
500
450
400
350
300
250
200
150
100
50
0
-50
-100
-150
150
200
250
300
350
400
450
500
550
600
650

Fig. 3

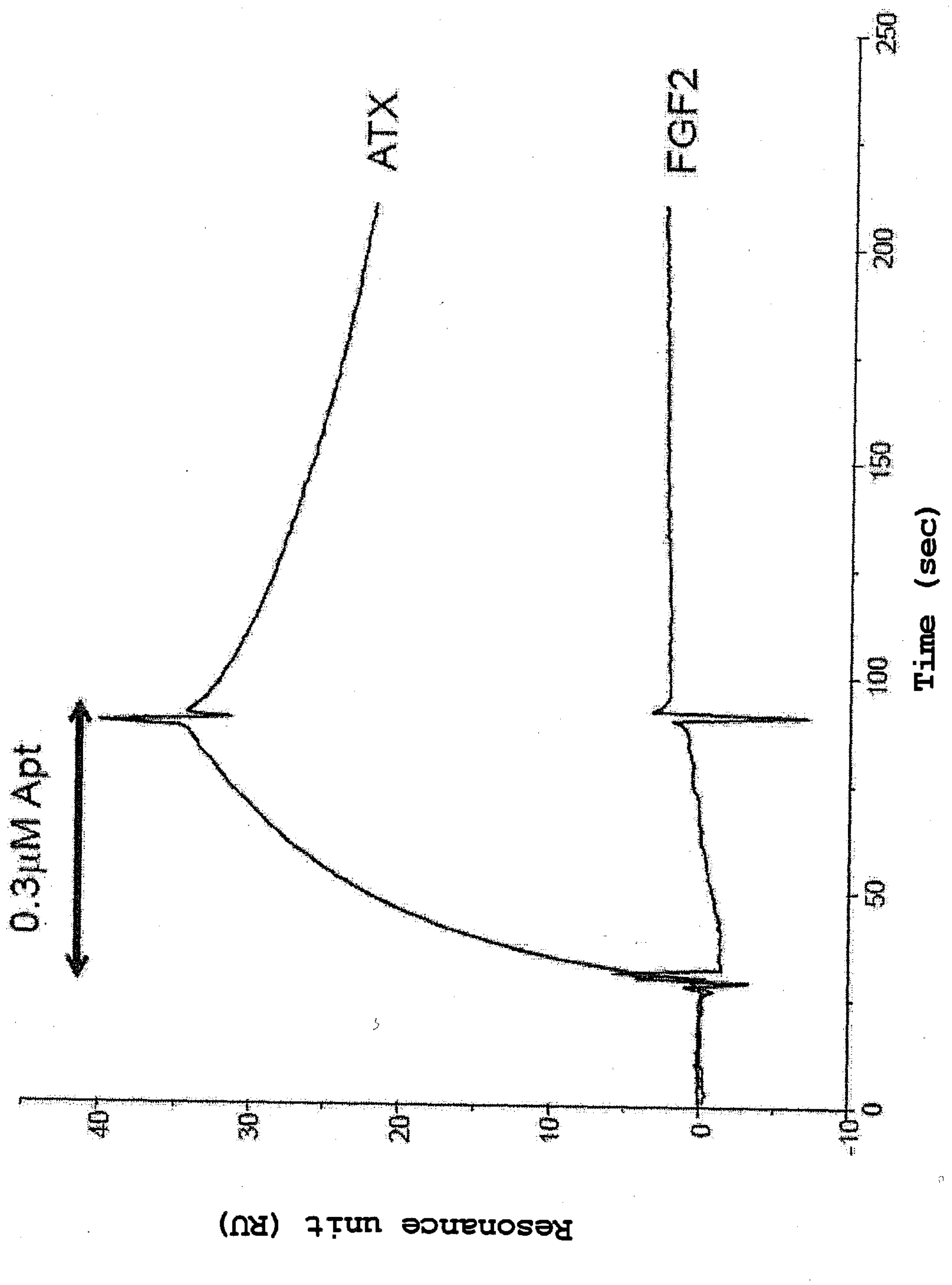
0.3μM Apt
ATX
FGF2
Resonance unit (RU)
Time (sec)
-10
0
10
20
30
40
0
50
100
150
200
250

Fig. 4

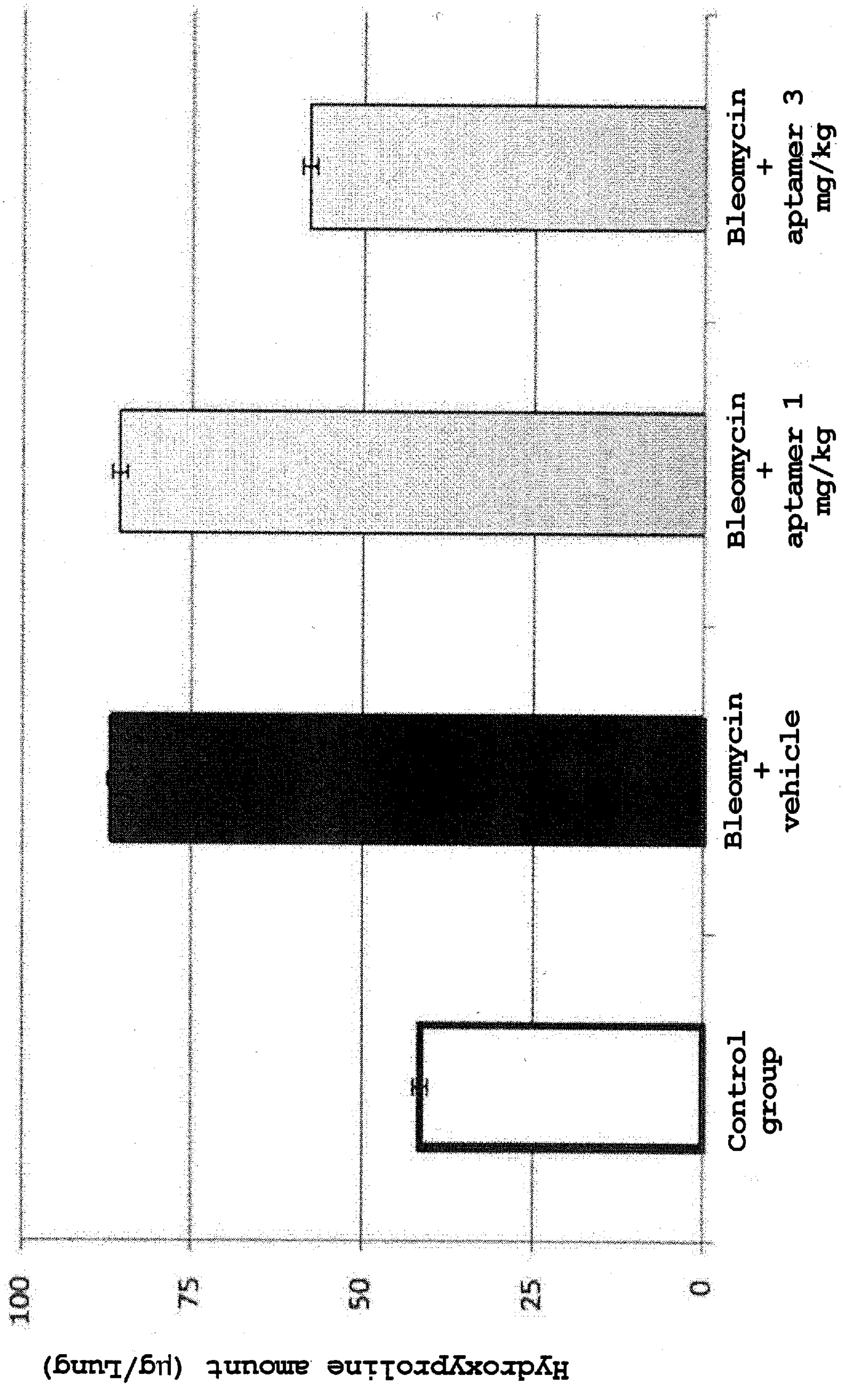
Hydroxyproline amount (μg/Lung)
100
75
50
25
0
Control group
Bleomycin + vehicle
Bleomycin + aptamer 1 mg/kg
Bleomycin + aptamer 3 mg/kg

Fig. 5

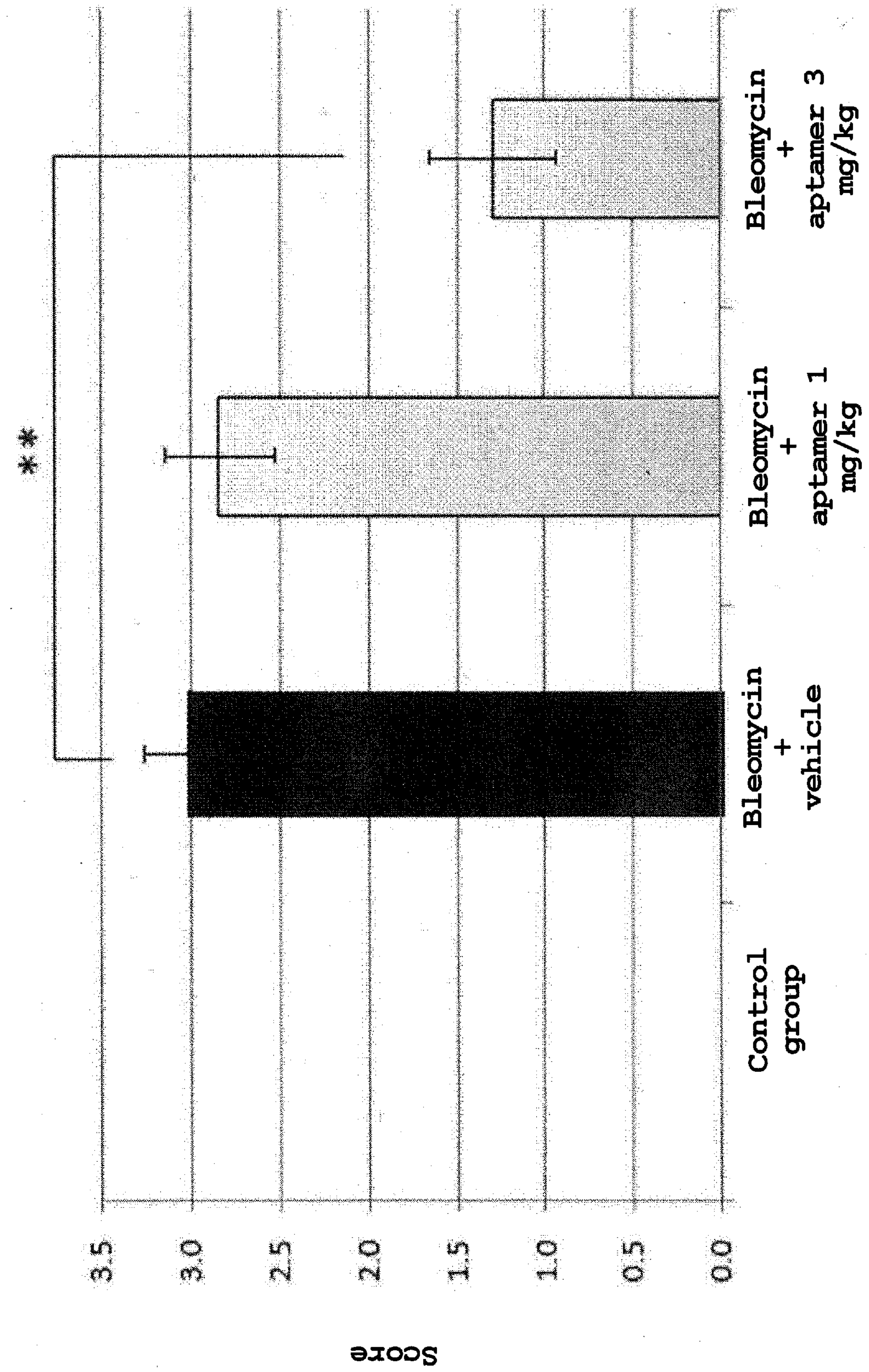
**
Score
3.5
3.0
2.5
2.0
1.5
1.0
0.5
0.0
Control group
Bleomycin + vehicle
Bleomycin + aptamer 1 mg/kg
Bleomycin + aptamer 3 mg/kg

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9119813 A
- WO 9408050 A
- WO 9507364 A
- US 5660985 A
- US 5756703 A
- JP 2014090755 A


### Non-patent literature cited in the description


- **SPROAT et al.** *Nucl. Acid. Res.,* 1991, vol. 19, 733-738
- **COTTON et al.** *Nucl. Acid. Res.,* 1991, vol. 19, 2629-2635
- **HOBBS et al.** *Biochemistry,* 1973, vol. 12, 5138-5145
- *Tetrahedron Lett.,* 1997, vol. 38, 8735-8738
- *Tetrahedron,* 2003, vol. 59, 5123-5128
- **RAHMAN S.M.A. ; SEKI S. ; OBIKA S. ; YOSHIKAWA H. ; MIYASHITA K. ; IMANISHI T.** *J. Am. Chem. Soc.,* 2008, vol. 130, 4886-4896
- Nucleic Acid. vol. 2
- Synthesis and Analysis of Nucleic Acid. Hirokawa Publishing Company
- **ELLINGTON et al.** *Nature,* 1990, vol. 346, 818-822
- **TUERK et al.** *Science,* 1990, vol. 249, 505-510
- **ELLINGTON ; SZOSTAK.** *Nature,* 1990, vol. 346, 818-822
- **TUERK ; GOLD.** *Science,* 1990, vol. 249, 505-510
- **M. ZUKER.** *Nucleic Acids Res.,* 2003, vol. 31 (13), 3406-3415

SEQUENCE LISTING

<110> RIBOMIC INC.

<120> Aptamer that binds to autotaxin and inhibits its physiological activity and use thereof

<130> 092336

<150> JP 2014-090755
<151> 2014-04-24

<160> 53

<170> PatentIn version 3.5

<210> 1
<211> 73
<212> DNA
<213> Artificial Sequence

<220>
<223> DNA template

<220>
<221> misc_feature
<222> (21)..(50)
<223> n is a, c, g, or t

<400> 1

**cggatacgtc tcacttcgtc nnnnnnnnnn nnnnnnnnnn nnnnnnnnnn cagagcgttt 60**

**agagtgcggt ccc 73**

<210> 2
<211> 40
<212> DNA
<213> Artificial Sequence

<220>
<223> Primer

<400> 2
taatacgact cactataggg accgcactct aaacgctctg 40

<210> 3
<211> 20
<212> DNA
<213> Artificial Sequence

<220>
<223> Primer

<400> 3
cggatacgtc tcacttcgtc 20

<210> 4
<211> 73
<212> RNA
<213> Artificial Sequence

<220>
<223> Aptamer

<400> 4

gggaccgcac ucuaaacgcu cugagggaaa cagguuuugc uccucggagc guggacgaag 60

ugagacguau ccg 73

<210> 5
<211> 81
<212> DNA
<213> Artificial Sequence

<220>
<223> DNA template

<220>
<221> misc_feature
<222> (20)..(59)
<223> n is a, c, g, or t

<400> 5

gtacgaagac gcatctcacn nnnnnnnnnn nnnnnnnnnn nnnnnnnnnn nnnnnnnnng 60

ttccatgtcc tgttgccacc c 81

<210> 6
<211> 39
<212> DNA
<213> Artificial Sequence

<220>
<223> Primer

<400> 6
taatacgact cactataggg tggcaacagg acatggaac 39

<210> 7
<211> 19
<212> DNA
<213> Artificial Sequence

<220>
<223> Primer

<400> 7
gtacgaagac gcatctcac 19

<210> 8
<211> 81
<212> RNA
<213> Artificial Sequence

<220>
<223> Aptamer

<400> 8

gggugcaac aggacaugga acgcgcccca acugcuugaa acagguuuug cugagcagug 60

augugagaug cgucuucgua c 81

<210> 9
<211> 81
<212> RNA
<213> Artificial Sequence

<220>
<223> Aptamer

<400> 9

gggugcaac aggacaugga acgcgcccca acugcuugaa acugguuuug cugagcagug 60

augugagaug cgucuucgua c 81

<210> 10
<211> 15
<212> RNA
<213> Artificial Sequence

<220>
<223> Consensus sequence

<400> 10
gaaacagguu uugcu 15

<210> 11
<211> 73
<212> DNA
<213> Artificial Sequence

<220>
<223> DNA template

<220>
<221> misc_feature
<222> (21)..(50)
<223> n is a, c, g, or t

<400> 11

aagcttcgta agtcgcagtc nnnnnnnnnn nnnnnnnnnn nnnnnnnnnn cagcgtagta 60

gtggtcctaa ccc 73

<210> 12
<211> 40
<212> DNA
<213> Artificial Sequence

<220>
<223> Primer

<400> 12
taatacgact cactataggg ttaggaccac tactacgctg 40

<210> 13
<211> 20
<212> DNA
<213> Artificial Sequence

<220>
<223> Primer

<400> 13
aagcttcgta agtcgcagtc 20

<210> 14
<211> 52
<212> RNA
<213> Artificial Sequence

<220>
<223> Aptamer

<400> 14
gggcuaaacg cucugaggga aacagguuuu gcuccucgga gcguggacgc cc 52

<210> 15
<211> 37
<212> RNA
<213> Artificial Sequence

<220>
<223> Aptamer

<400> 15
ccgcucugag ggaaacaggu uuugcuccuc ggagcgg 37

<210> 16
<211> 29
<212> RNA
<213> Artificial Sequence

<220>
<223> Aptamer

<400> 16
ucugagggaa acagguuuug cuccucgga 29

<210> 17
<211> 25
<212> RNA
<213> Artificial Sequence

<220>
<223> Aptamer

<400> 17
ugagggaaac agguuuugcu ccucg 25

<210> 18
<211> 27
<212> RNA
<213> Artificial Sequence

<220>
<223> Aptamer

<400> 18
cugagggaaa cagguuuugc uccucgg 27

<210> 19
<211> 25
<212> RNA
<213> Artificial Sequence

<220>
<223> Aptamer

<400> 19
cgagggaaac agguuuugcu ccucg 25

<210> 20
<211> 23
<212> RNA
<213> Artificial Sequence

<220>
<223> Aptamer

<400> 20
gagggaaaca gguuuugcuc cuc 23

<210> 21
<211> 21
<212> RNA
<213> Artificial Sequence

<220>
<223> Aptamer

<400> 21
agggaaacag guuuugcucc u 21

<210> 22
<211> 19
<212> RNA
<213> Artificial Sequence

<220>
<223> Aptamer

<400> 22
gggaaacagg uuuugcucc 19

<210> 23
<211> 17
<212> RNA

<213> Artificial Sequence

<220>
<223> Aptamer

<400> 23
ggaaacaggu uuugcuc 17

<210> 24
<211> 29
<212> RNA
<213> Artificial Sequence

<220>
<223> Aptamer

<400> 24
acugcuugaa acagguuuug cugagcagu 29

<210> 25
<211> 69
<212> DNA
<213> Artificial Sequence

<220>
<223> DNA template

<400> 25

gggcgtccac gctccgagga gcaaaacctg tttccctcag agcgtttagc cctatagtga 60

gtcgtatta 69

<210> 26
<211> 29
<212> RNA
<213> Artificial Sequence

<220>
<223> Aptamer

<400> 26
ucugagggua acagguuaug cuccucgga 29

<210> 27
<211> 29
<212> RNA
<213> Artificial Sequence

<220>
<223> Aptamer

<400> 27
ucugagggau acagguauug cuccucgga 29

<210> 28
<211> 29

<212> RNA
<213> Artificial Sequence

<220>
<223> Aptamer

<400> 28
ucugagggaa ucaggauuug cuccucgga 29

<210> 29
<211> 29
<212> RNA
<213> Artificial Sequence

<220>
<223> Aptamer

<400> 29
uccgagggaa acagguuuug cuccucgga 29

<210> 30
<211> 29
<212> RNA
<213> Artificial Sequence

<220>
<223> Aptamer

<400> 30
ucugagggaa acagguuuug cuccucaga 29

<210> 31
<211> 29
<212> RNA
<213> Artificial Sequence

<220>
<223> Aptamer

<400> 31
ucugagggaa acaggcuuug cuccucgga 29

<210> 32
<211> 29
<212> RNA
<213> Artificial Sequence

<220>
<223> Aptamer

<400> 32
ucugagggaa acagguuucg cuccucgga 29

<210> 33
<211> 29
<212> RNA
<213> Artificial Sequence

<220>
<223> Aptamer

<400> 33
ucugagggga acagguuuug cuccucgga 29

<210> 34
<211> 29
<212> RNA
<213> Artificial Sequence

<220>
<223> Aptamer

<400> 34
ucugagggag acagguuuug cuccucgga 29

<210> 35
<211> 29
<212> RNA
<213> Artificial Sequence

<220>
<223> Aptamer

<400> 35
ucugagggaa gcagguuuug cuccucgga 29

<210> 36
<211> 28
<212> RNA
<213> Artificial Sequence

<220>
<223> Aptamer

<400> 36
ucugagggaa acagguuugc uccucgga 28

<210> 37
<211> 28
<212> RNA
<213> Artificial Sequence

<220>
<223> Aptamer

<400> 37
ucugagggaa cagguuuugc uccucgga 28

<210> 38
<211> 73
<212> DNA
<213> Artificial Sequence

<220>
<223> DNA template

<400> 38

cggatacgtc tcacttcgtc cacgctccga ggagcaaaac ctgtttccct cagagcgttt 60

agagtgcggt ccc 73

<210> 39
<211> 36
<212> DNA
<213> Artificial Sequence

<220>
<223> Primer

<400> 39
taatacgact cactataggg accgcactct aaacgc 36

<210> 40
<211> 29
<212> RNA
<213> Artificial Sequence

<220>
<223> Aptamer

<400> 40
ucugagggua acagguuuug cuccucgga 29

<210> 41
<211> 29
<212> RNA
<213> Artificial Sequence

<220>
<223> Aptamer

<400> 41
ucugagggaa acaggucuug cuccucgga 29

<210> 42
<211> 15
<212> RNA
<213> Artificial Sequence

<220>
<223> Consensus sequence

<400> 42
gwaacagguu uugcu 15

<210> 43
<211> 73
<212> DNA
<213> Artificial Sequence

<220>
<223> DNA template

<400> 43

**cggatacgtc tcacttcgtc cacgctccga ggagcaaaac ctgtttccct cagagcgttt 60**

**agagtgcggt ccc 73**

<210> 44
<211> 29
<212> RNA
<213> Artificial Sequence

<220>
<223> Aptamer

<400> 44
ucugggggaa acagguuuug cuccccgga 29

<210> 45
<211> 29
<212> RNA
<213> Artificial Sequence

<220>
<223> Aptamer

<400> 45
ucugagggaa acagguuuug cuccucgaa 29

<210> 46
<211> 29
<212> RNA
<213> Artificial Sequence

<220>
<223> Aptamer

<400> 46
ucugagggaa acagguucug cuccucgga 29

<210> 47
<211> 29
<212> RNA
<213> Artificial Sequence

<220>
<223> Aptamer

<400> 47
uccgagggaa acagguuuug cuccucgca 29

<210> 48
<211> 28
<212> RNA
<213> Artificial Sequence

<220>
<223> Aptamer

<400> 48
ucugaggaaa cagguuuugc ugcucgga 28

<210> 49
<211> 15
<212> RNA
<213> Artificial Sequence

<220>
<223> Consensus sequence

<400> 49
gwaacagguy yygcu 15

<210> 50
<211> 30
<212> RNA
<213> Artificial Sequence

<220>
<223> Aptamer

<400> 50
uagagaugga aacagguuuu gcucaucuca 30

<210> 51
<211> 29
<212> RNA
<213> Artificial Sequence

<220>
<223> Aptamer

<400> 51
uuugaaggaa acagguuuug cucuucgag 29

<210> 52
<211> 29
<212> RNA
<213> Artificial Sequence

<220>
<223> Aptamer

<400> 52
uucgagggaa acagguuuug cuccucaaa 29

<210> 53
<211> 29
<212> RNA
<213> Artificial Sequence

<220>
<223> Aptamer

<400> 53
ugggaaggaa acagguuuug cucuucacc 29